Europäisches Patentamt

European Patent Office

Office européen des brevets

(19)

(11) Veröffentlichungsnummer: **0 364 942 B1**

(12) **EUROPÄISCHE PATENTSCHRIFT**

(45) Veröffentlichungstag der Patentschrift: **22.02.95**

(51) Int. Cl.⁶: **C07K 14/815**

(21) Anmeldenummer: **89119233.8**

(22) Anmeldetag: **17.10.89**

(54) **Neue Isohirudine.**

(30) Priorität: **21.10.88 DE 3835815**

(43) Veröffentlichungstag der Anmeldung:
**25.04.90 Patentblatt 90/17**

(45) Bekanntmachung des Hinweises auf die
Patenterteilung:
**22.02.95 Patentblatt 95/08**

(84) Benannte Vertragsstaaten:
**AT BE CH DE ES FR GB GR IT LI LU NL SE**

(56) Entgegenhaltungen:
**WO-A-86/03493**

**FEBS LETTERS, Band 255, Nr. 1, September
1989, Seiten 105-110, Elsevier SciencePublishers B.V. (Biomedical Division), Amsterdam,
NL; M. SCHARF et al.:"Primary structures of
new "iso-hirudins""**

**DIE PHARMAZIE, Band 36, Nr. 10, Okfober
1981, Seiten 653-600; P. WALSMANN etal.:
"Biochemische und pharmakologische
Aspekfe des Thrombininhibitors Hirudin"**

**Chang J.-Y., FEBS, Vol.164(2), pp.307-13
(1983)**

**Harvey et al., Proc.Natl.Acad.Sci. USA,
Vol.83, pp.1084-88 (1986)**

(73) Patentinhaber: **HOECHST AKTIENGESELL-
SCHAFT**

**D-65926 Frankfurt (DE)**

(72) Erfinder: **Tripier, Dominique, Dr.
Im Kirschgarten 16
D-6239 Eppstein/Taunus (DE)**
Erfinder: **Crause, Peter, Dr.
Schopenhauerstrasse 31
D-6050 Offenbach (DE)**
Erfinder: **Habermann, Paul, Dr.
Rossertstrasse 35
D-6239 Eppstein/Taunus (DE)**
Erfinder: **Kramer, Martin, Prof. Dr.
Fichtestrasse 4
D-6200 Wiesbaden (DE)**
Erfinder: **Engels, Joachim, Prof. Dr.
Feldbergstrasse 1
D-6242 Kronberg/Taunus (DE)**
Erfinder: **Scharf, Matthias
Reuterweg 76
D-6000 Frankfurt am Main (DE)**

EP 0 364 942 B1

**Beschreibung**

Die Erfindung betrifft neue Thrombininhibitoren aus der Familie der Hirudine, die sich durch Mutation in der Proteinkette von den bisher bekannten Hirudinen unterscheiden.

Hirudine sind z.B. bekannt aus EP-A 142 860; EP-A 158 564; EP-A 158 986; EP-A 168 342; EP-A 171 024; EP-A 193 175; EP-A 200 655; EP-A 209 061; Chang, FEBS, Bd. 164 (1983) 307; J. Dodt et al., Biol. Chem. Hoppe-Seyler 367 (1986) 803-811 und D. Tripier, Folia Haematol. Leipzig 115 (1988) 1-2, 30-35. Diese Hirudine können jedoch teilweise über aktive Zentren an Träger oder über Aminosäuren, die an der Bindung zu Thrombin beteiligt sind, gebunden sein. Ferner haben die bekannten Hirudine geringe transdermale Eigenschaften und sind aufgrund hoher Eliminationsraten sehr kurzlebig, so daß eine stationäre oder ambulante Thromboseprophylaxe erschwert ist.

Es stellte sich daher die Aufgabe, Hirudine mit hoher spezifischer Aktivität, hoher Stabilität und guter Pharmakokinetik zu finden. Ferner sollten sich diese Hirudine bei der Fixierung auf Trägern durch bessere chemische Handhabbarkeit auszeichnen.

Diese Aufgabe wird erfindungsgemäß gelöst durch die Isohirudine der Formel (A)

```
    1                    5                           10
    A - B - Tyr - Thr - Asp - Cys - Thr - Glu - Ser - Gly -
                         15                          20
    C - E - Leu - Cys - Leu - Cys - F - G - Ser - I - Val -
                    25                          30
    Cys - Gly - J - Gly - Asn - Lys - Cys - K - Leu - Gly -


                    35                          40
    Ser - L - Gly - Glu - M - Asn - N - Cys - Val - Thr - Gly -
               45                          50
    Glu - Gly - Thr - Pro - Lys - Pro - Gln - Ser - His - Asn -
               55                          60
    Asp - Gly - Asp - Phe - Glu - Glu - Ile - Pro - Glu - Glu -
               65
    Tyr(R) - Leu - Gln

                                                    (A)
```

in welcher A Val, B Val, C Gln, E Asn, F Glu, G Gly, I Asn, J Lys, K Ile, L Asp, M Lys, N Gln und R Wasserstoff oder SO₃H bedeuten, oder

in welcher A Val, B Val, C Gln, E Asn, F Glu, G Gly, I Asn, J Asn, K Ile, L Asp, M Lys, N Gln und R Wasserstoff oder SO₃H bedeuten, oder

in weicher A Ile, B Thr, C Gln, E Asn, F Glu, G Gly, I Asn, J Asn, K Lys, L Asp, M Glu, N Gln und R Wasserstoff oder SO₃H bedeuten, oder

in weicher A Ile, B Thr, C Gln, E Asp, F Glu, G Gly, I Asn, J Lys, K Ile, L Asn, M Glu, N Gln und R Wasserstoff oder SO₃H bedeuten, oder

in weicher A Ile, B Thr, C Gln, E Asp, F Glu, G Gly, I Asp, J Lys, K Ile, L Asn, M Glu, N Gln und R Wasserstoff oder SO₃H bedeuten, oder

in weicher A Ile, B Thr, C Gln, E Asn, F Glu, G Gly, I Asn, J Lys, K Ile, L Asn, M Glu, N Gln und R Wasserstoff oder SO₃H bedeuten,

2

EP 0 364 942 B1

oder
in welcher A Val, B Val, C Gln, E Asn, F Glu, G Asp, I Asn, J Glu, K Ile, L Asn, M Lys, N Gln und R Wasserstoff oder $SO_3H$ bedeuten,
oder
in welcher A Val, B Val, C Glu, E Asp, F Glu, G Gly, I Asn, J Glu, K Ile, L Asp, M Lys, N Glu und R Wasserstoff oder $SO_3H$ bedeuten,
oder
in welcher A Val, B Val, C Gln, E Asn, F Glu, G Asp, I Asn, J Gln, K Ile, L Asn, M Lys, N Gln und R Wasserstoff oder $SO_3H$ bedeuten,
oder
in welcher A Val, B Val, C Gln, E Asn, F Gln, G Asp, I Asn, J Gln, K Ile, L Asn, M Lys, N Gln und R Wasserstoff oder $SO_3H$ bedeuten,
oder
in welcher A Val, B Val, C Gln, E Asn, F Gln, G Gly, I Asn, J Gln, K Ile, L Asn, M Lys, N Gln und R Wasserstoff oder $SO_3H$ bedeuten.

Insbesondere seien die folgenden Isohirudine genannt, wobei R Wasserstoff oder $SO_3H$ bedeutet:

```
Val - Val - Tyr - Thr - Asp - Cys - Thr - Glu - Ser -
Gly - Gln - Asn - Leu - Cys - Leu - Cys - Glu - Gly -
Ser - Asn - Val - Cys - Gly - Lys - Gly - Asn - Lys -
Cys - Ile - Leu - Gly - Ser - Asp - Gly - Glu - Lys -      (Ia)
Asn - Gln - Cys - Val - Thr - Gly - Glu - Gly - Thr -
Pro - Lys - Pro - Gln - Ser - His - Asn - Asp - Gly -
Asp - Phe - Glu - Glu - Ile - Pro - Glu - Glu - Tyr(R) -
Leu - Gln
```

EP 0 364 942 B1

```
Val - Val - Tyr - Thr - Asp - Cys - Thr - Glu - Ser -
Gly - Gln - Asn - Leu - Cys - Leu - Cys - Glu - Gly -
Ser - Asn - Val - Cys - Gly - Asn - Gly - Asn - Lys -
Cys - Ile - Leu - Gly - Ser - Asp - Gly - Glu - Lys -        (I)
Asn - Gln - Cys - Val - Thr - Gly - Glu - Gly - Thr -
Pro - Lys - Pro - Gln - Ser - His - Asn - Asp - Gly -
Asp - Phe - Glu - Glu - Ile - Pro - Glu - Glu - Tyr(R) -
Leu - Gln


Ile - Thr - Tyr - Thr - Asp - Cys - Thr - Glu - Ser -
Gly - Gln - Asn - Leu - Cys - Leu - Cys - Glu - Gly -
Ser - Asn - Val - Cys - Gly - Asn - Gly - Asn - Lys -
Cys - Lys - Leu - Gly - Ser - Asp - Gly - Glu - Glu -       (IIa)
Asn - Gln - Cys - Val - Thr - Gly - Glu - Gly - Thr -
Pro - Lys - Pro - Gln - Ser - His - Asn - Asp - Gly -
Asp - Phe - Glu - Glu - Ile - Pro - Glu - Glu - Tyr(R) -
Leu - Gln


Ile - Thr - Tyr - Thr - Asp - Cys - Thr - Glu - Ser -
Gly - Gln - Asp - Leu - Cys - Leu - Cys - Glu - Gly -
Ser - Asn - Val - Cys - Gly - Lys - Gly - Asn - Lys -
Cys - Ile - Leu - Gly - Ser - Asn - Gly - Glu - Glu -       (II)
Asn - Gln - Cys - Val - Thr - Gly - Glu - Gly - Thr -
Pro - Lys - Pro - Gln - Ser - His - Asn - Asp - Gly -
Asp - Phe - Glu - Glu - Ile - Pro - Glu - Glu - Tyr(R) -
Leu - Gln


Ile - Thr - Tyr - Thr - Asp - Cys - Thr - Glu - Ser -
Gly - Gln - Asp - Leu - Cys - Leu - Cys - Glu - Gly -
Ser - Asp - Val - Cys - Gly - Lys - Gly - Asn - Lys -
Cys - Ile - Leu - Gly - Ser - Asn - Gly - Glu - Glu -       (II')
Asn - Gln - Cys - Val - Thr - Gly - Glu - Gly - Thr -
Pro - Lys - Pro - Gln - Ser - His - Asn - Asp - Gly -
Asp - Phe - Glu - Glu - Ile - Pro - Glu - Glu - Tyr(R) -
Leu - Gln
```

4

```
Ile - Thr - Tyr - Thr - Asp - Cys - Thr - Glu - Ser -
Gly - Gln - Asn - Leu - Cys - Leu - Cys - Glu - Gly -
Ser - Asn - Val - Cys - Gly - Lys - Gly - Asn - Lys -
Cys - Ile - Leu - Gly - Ser - Asn - Gly - Glu - Glu -      (IIb)
Asn - Gln - Cys - Val - Thr - Gly - Glu - Gly - Thr -
Pro - Lys - Pro - Gln - Ser - His - Asn - Asp - Gly -
Asp - Phe - Glu - Glu - Ile - Pro - Glu - Glu - Tyr(R) -
Leu - Gln
```

```
Val - Val - Tyr - Thr - Asp - Cys - Thr - Glu - Ser -
Gly - Gln - Asn - Leu - Cys - Leu - Cys - Glu - Asp -
Ser - Asn - Val - Cys - Gly - Glu - Gly - Asn - Lys -
Cys - Ile - Leu - Gly - Ser - Asn - Gly - Glu - Lys -      (IIIa)
Asn - Gln - Cys - Val - Thr - Gly - Glu - Gly - Thr -
Pro - Lys - Pro - Gln - Ser - His - Asn - Asp - Gly -
Asp - Phe - Glu - Glu - Ile - Pro - Glu - Glu - Tyr(R) -
Leu - Gln
```

```
Val - Val - Tyr - Thr - Asp - Cys - Thr - Glu - Ser -
Gly - Glu - Asp - Leu - Cys - Leu - Cys - Glu - Gly -
Ser - Asn - Val - Cys - Gly - Glu - Gly - Asn - Lys -
Cys - Ile - Leu - Gly - Ser - Asp - Gly - Glu - Lys -      (IIIa')
Asn - Glu - Cys - Val - Thr - Gly - Glu - Gly - Thr -
Pro - Lys - Pro - Gln - Ser - His - Asn - Asp - Gly -
Asp - Phe - Glu - Glu - Ile - Pro - Glu - Glu - Tyr(R) -
Leu - Gln
```

```
Val - Val - Tyr - Thr - Asp - Cys - Thr - Glu - Ser -
Gly - Gln - Asn - Leu - Cys - Leu - Cys - Glu - Asp -
Ser - Asn - Val - Cys - Gly - Gln - Gly - Asn - Lys -
Cys - Ile - Leu - Gly - Ser - Asn - Gly - Glu - Lys -      (III)
Asn - Gln - Cys - Val - Thr - Gly - Glu - Gly - Thr -
Pro - Lys - Pro - Gln - Ser - His - Asn - Asp - Gly -
Asp - Phe - Glu - Glu - Ile - Pro - Glu - Glu - Tyr(R) -
Leu - Gln
```

Val - Val - Tyr - Thr - Asp - Cys - Thr - Glu - Ser -
Gly - Gln - Asn - Leu - Cys - Leu - Cys - Gln - Asp -
Ser - Asn - Val - Cys - Gly - Gln - Gly - Asn - Lys -
Cys - Ile - Leu - Gly - Ser - Asn - Gly - Glu - Lys -    (IIIb)
Asn - Gln - Cys - Val - Thr - Gly - Glu - Gly - Thr -
Pro - Lys - Pro - Gln - Ser - His - Asn - Asp - Gly -
Asp - Phe - Glu - Glu - Ile - Pro - Glu - Glu - Tyr(R) -
Leu - Gln


Val - Val - Tyr - Thr - Asp - Cys - Thr - Glu - Ser -
Gly - Gln - Asn - Leu - Cys - Leu - Cys - Gln - Gly -
Ser - Asn - Val - Cys - Gly - Gln - Gly - Asn - Lys -
Cys - Ile - Leu - Gly - Ser - Asn - Gly - Glu - Lys -    (IIIb')
Asn - Gln - Cys - Val - Thr - Gly - Glu - Gly - Thr -
Pro - Lys - Pro - Gln - Ser - His - Asn - Asp - Gly -
Asp - Phe - Glu - Glu - Ile - Pro - Glu - Glu - Tyr(R) -
Leu - Gln

Die Erfindung betrifft ferner ein Verfahren zur Herstellung von Isohirudinen, das dadurch gekennzeichnet ist, daß man Isohirudine aus Blutegeln mit Hilfe einer Kombination von Extraktionsmethoden, Fällungsmethoden und chromatographischen Verfahren isoliert und reinigt, eine gegebenenfalls vorhandene Phenolestergruppe R gewünschtenfalls hydrolytisch unter Bildung der phenolischen Hydroxylgruppe abspaltet und die erhaltenen Polypeptide gegebenenfalls in ihre physiologisch verträglichen Salze überführt.

Die Isohirudine werden vorzugsweise zunächst wie von F. Markwardt in Biomed. Biochem. Acta 44 (1985) 1007-1013 oder in EP-A 158 986 und EP-A 209 061 beschrieben extrahiert und anschließend die Roh-Isohirudine zur Präzipitation gebracht. Die weitere Isolierung und Reinigung erfolgt mittels einer Kombination von chromatographischen Verfahren, wie z.B. von P. Walsmann in Pharmazie 36 (1981) 860-861, beschriebenen. Bevorzugt wird jedoch folgende Kombination von Chromatographieverfahren angewendet:

Ionenaustauschchromatographie → Gelpermeationschromatographie → Affinitätschromatographie → Gelpermeationschromatographie → Ionenaustauschchromatographie → Micro-Bore RP-HPLC.

Die einzelnen Chromatographieverfahren sind für sich allgemein bekannt.

Das erfindungsgemäße Verfahren zeichnet sich durch die Kombination der verschiedenen Chromatographieverfahren einschließlich der abschließenden Micro-Bore RP-HPLC aus, bei der Säulen von 1 mm Innendurchmesser verwendet werden. Bedingt durch eine niedrigere Bodenhöhe und die geringere Diffusion in der Säule resultiert hierbei eine höhere Auflösung als bei herkömmlichen 4,6 mm-Säulen (R.P.W. Scott, J. Chromatographie Science 23 (1986) 233-237; R. Gill, J. Chromatographie 16 (1986) 281).

Die hier verwendete Micro-Bore RP-HPLC-Anlage besteht aus zwei Beckman 114 M Solvent Delivery Modulen, die von einem 421 a Controller gesteuert werden. Die Pufferleitung zu jeder Pumpe - aus passiviertem 1/8 Zoll V8 Stahlrohr - ist ber ein T-Stück mit einem dynamischen Mikrogradientenmischer verbunden, der die Pufferlösungen homogenisiert, wobei die resultierenden Pulsationen durch einen on-line eingeschalteten Pulsationsdämpfer (Waters part Nr. 98060) abgefangen werden und die Pufferlösung über das Reodyne-Ventil eines Probengebers (Gilson Abimed Model 231, Dilutor Modell 401) in die Micro-Bore Säule eingeleitet wird. Die mit Aquapore RP-300 (300 Å pore-size) 7 $\mu$m spherical Silica gefüllte Micro-Bore-Säule (Applied Biosystems Kat. Nr. 400422 Rp) liegt in einem Ofen (Sykam S 4110, Temperatur 40 °C). Detektiert wird bei 205 nm in einem UV-Detektor (Sykam S 3300, der auf 0,08 AUFS ausgerüstet und eingestellt ist. Die Registrierung erfolgt mittels eines Kompensationsschreibers (10 mv FS), die Integration mit dem LAS-System (Hewlett Packard) nach A/D Konversion (HP 18625 A A/D Convertor). Ein linearer Gradientenbetrieb wird optimiert.

Folgende Mischungen A und B werden als Puffer verwendet:

A: 90 Gew.-% Wasser, 10 Gew.-% Acetonitril (AcCN) + 0,1 Vol.-% Trifluoressigsäure (TFA)

B: 90 Gew.-% AcCN, 10 Gew.-% Wasser + 0,1 Vol.-% TFA.

Dabei werden 3 unterschiedliche Gradienten für die verschiedenen Trenn- und Reinigungsschritte benutzt:

a) für die Trennung der Isohirudine untereinander in analytischem und präparativem Betrieb:

0 % B bis 40 % B (Steilheit 0,06 % B/min)

b) für Rechromatographie und Endreinigung:

0 % B bis 35 % B (Steilheit 0,4 % B/min)

c) für die Peptidtrennung:

0 % B bis 30 % B (Steilheit 1,7 % B/min)

Es wird bei einer Flußrate von 80 $\mu$l/min gearbeitet und manuell fraktioniert.

Durch den erfindungsgemäßen Einsatz der Micro-Bore RP-HPLC - zusätzlich zu den bekannten Isolierungs- und Reinigungsverfahren - ist es erst möglich, weitere neue Peptidtrennungen durchzuführen. Leider treten aber immer noch Fälle auf, bei denen aufgrund einer zu großen Verwandtschaft der Einzelkomponenten auch hier eine Trennung unvollständig bleibt. Z.B. ist dies der Fall bei den Isohirudinen II und II'; IIIa, IIIa'; IIIb und IIIb'. Der Zusatz ' bedeutet also, daß diese Mutationen während der Sequenzanalyse der verwandten Proteine gefunden wurden, d.h. mitsequenziert wurden. So wurde z.B. bei der Analyse des Isohirudins II in der Abbaustufe 20 das Isohirudin II' gefunden.

Die in dieser Anmeldung beschriebenen neuen Isohirudine werden zunächst nach aus der Literatur bekannten Methoden gereinigt, einschließlich der Affinitätschromatographie an Thrombin-Sepharose-Säulen. Danach wird das resultierende Isohirudingemisch mit Hilfe der Micro-Bore RP-HPLC in seine Komponenten getrennt. Bei schwierigen Trennproblemen werden die isolierten Fraktionen unter leicht veränderten Bedingungen rechromatographiert. Jedes neue Isohirudin wird bis zur Homogenität oder Quasi-Homogenität gereinigt und in seiner Sequenz vollkommen aufgeklärt, wobei in einigen Fällen nicht nur die Aminosäuresequenz durch Sequenzanalyse ermittelt wird, sondern zusätzlich proteinchemische Methoden, wie spezifische chemische Spaltungen, verwendet werden, um die neuen Sequenzen zu belegen.

Die Aminosäureanalyse wird mit einem Aminosäureanalyser Beckmann 6300 nach der Anleitung des Herstellers durchgeführt. Dazu wurden die Proteine (30-50 pmol) zuvor in einem 4 x 40 mm Quarz-Reagenzglas getrocknet und anschließend in der Dampfphase mit azeotropischem HCl/0,8 % Phenol unter Stickstoffatmosphäre bei 110 °C hydrolysiert. Für jeden Ansatz werden 500 pmol Insulin-Standard mitanalysiert. Integriert wird mit dem LAS-System (Hewlett Packard).

Durch automatisierte Sequenzanalyse am native Protein wird die N-terminale Aminosäuresequenz bestimmt. Es wird mit einem 477 A Pulsed liquid-phase (TM) Protein Sequencer der Fa. Applied Biosystems nach den Angaben des Herstellers gearbeitet, der mit einem 120 A PTH-Analysator mit Datenauswertsystem on-line gekoppelt ist.

In einigen Fällen, d.h. für die Isohirudine I, II, IIb, III und IIIa, wird zusätzlich eine Protease-spezifische Peptidspaltung durchgeführt. Dazu werden zunächst die "Disulfidbrücken" der jeweiligen Proteine mit Perameisensäure oxydiert, wobei Cystin in Cysteinsäure umgewandelt wird. Diese Oxidation bewirkt daß die Proteine ihre räumliche Konfiguration verlieren und ein Polymer (ein Knäul) von Aminosäuren ohne fest definierbare Konfiguration bilden. Nach der Oxidation wird das Reaktionsgemisch gefriergetrocknet, daß erhaltene Protein in einem geeigneten Puffer mit Trypsin gespalten, das Peptidgemisch mittels Micro-Bore RP-HPLC in die einzelnen Komponenten getrennt und in präparativer Arbeitsweise isoliert. Anschließend wird durch Aminosäureanalyse dieser Peptide die primäre Sequenz bestimmt. Für die spezifische Spaltung wird bevorzugt Trypsin, eine Serinprotease, eingesetzt, die nach einer basischen Aminosäure, d.h. nach Arg oder Lys, spaltet.

Die neuen Isohirudine der Formel A können außerdem analog dem in der EP-A 171 024 beschriebenen Verfahren hergestellt werden.

Die erfindungsgemäßen Isohirudine sind spezifische Thrombininhibitoren. Die quantitative Thrombin-Hemmung durch die erfindungsgemäßen Inhibitoren zeigte, daß der Komplex Thrombininhibitor/Thrombin praktisch nicht dissoziiert.

Mit Hilfe des nachfolgend beschriebenen Thrombinhemmtests kann während der Aufarbeitung und Reinigung die Aktivität und damit der Reinheitsgrad der erfindungsgemäßen Isohirudine bestimmt werden. Die so gereinigten Isohirudine der allgemeinen Formel (A) weisen dabei eine Thrombinhemmung von 12-16 AT-U/$\mu$g (Antithrombine Units/$\mu$g) auf.

**Thrombinhemmtest zur Aktivitätsbestimmung**

Der Test wird bei einem Gesamtvolumen von 200 $\mu$l in 96-Well-Mikrotiterplatten bei Raumtemperatur durchgeführt. Nach einer Präinkubation des Thrombins (Rinderthrombin 50 NIH-U/mg (Fa. Merck, Art.-Nr.

12374); Stammlösung: 40 NIH-U/ml in 20 mM MES pH 6, 0,154 mM NaCl, 0,2 % PEG 6000, 1 % BSA) in einem Endvolumen von 100 µl mit 1 bis 50 µl Isohirudin erfolgt der Reaktionsstart durch Substratzugabe. Da die Aktivitäten im Meßbereich des Tests zwischen 0,03 und 0,15 AT-U liegen, müssen die zu bestimmenden Proben entsprechend mit einem Testpuffer verdünnt werden.

Die erforderliche Menge Testpuffer (50 mM Tris/HCl, pH 8; 154 mM NaCl; 0,2 % Polyethylenglykol 6000) pro Well-Mikrotiterplatte wird vorgelegt. Anschließend werden je 50 µl 4 NIH-U/ml Thrombinlösung (siehe oben) und die Probe zugesetzt. Nach einer Inkubationszeit von 10 Minuten wird anschließend die Reaktion durch Zugabe von 100 µl 1mM Chromozym TH (Fa. Boehringer, Art.-Nr. 206849; 10 mM in Wasser/HCl, pH 6; Gebrauchslösung: 1 mM mit Testpuffer verdünnt) gestartet. Nach 5-10 minütiger Inkubation des Rest-Thrombins mit dem Chromogen-Substrat wird dann das freigesetzte Nitroanilin photometrisch bei 405 nm bestimmt. Ein Reaktionsstop ist nicht erforderlich, sofern ein ®ELISA-Reader (Easy-Reader EAR 400 (SLT-Labinstruments, Austria) für die schnelle Ablesung der Well-Mikrotiterplatten zur Verfügung steht. Ein Hirudinstandard ist im Prinzip auch nicht erforderlich, da durch den Einsatz einer definierten Thrombin-Aktivität eine absolute Bestimmung der Antithrombin-Units möglich ist. Allerdings führt die allmähliche Inaktivierung der Gebrauchslösung zu einem systematischen Fehler, der eliminiert werden kann, wenn man die Meßwerte auf einen definierten Hirudinstandard bezieht. Der Test wird dann unabhängig von der absoluten Thrombinkonzentration und erlaubt reproduzierbare Bestimmungen.

Folgende Kontrollen werden verwendet:

a) Blank
Die Thrombinlösung wird durch einen Puffer ersetzt (Photometernullabgleich)
b) Thrombin-Wert
Eine Well-Mikrotiterplatte wird ohne Inhibitor belassen.
c) Hirudinstandard
Auf einer Well-Mikrotiterplatte wird 0,07-0,1 AT-U eines bekannten Hirudinstandards (Hirudin Pentapharm "purified by affinity chromatography", spezifische Aktivität: 10 AT-U/µg, Konzentration: 0,1 AT-U/50 µl) zugesetzt.
Um die Konzentrationen der Probe festzustellen, wurde für die Micro-Bore HPLC eine Eichkurve für 1 bis 20 µg Isohirudin ermittelt.

Die nachfolgende Tabelle 1 stellt die auf der Micro-Bore HPLC ermittelten Konzentrationswerte denen nach dem Thrombinhemmtest ermittelten gegenüber:

Tabelle 1

| Isohirudin | Konzentration [µg/ml] | | AT-U/µg |
|---|---|---|---|
| | Micro-Bore HPLC | Thrombinhemmtest | |
| Ia | 56 | 59 | 14 |
| I | 56 | 62 | 15 |
| Ib | 34 | 38 | 16 |
| IIa | 68 | 72 | 15 |
| II | 120 | 140 | 16 |
| IIb | 137 | 145 | 16 |
| IIIa | 84 | 87 | 12 |
| III | 397 | 533 | 16 |
| IIIb | 97 | 109 | 14 |

Die Erfindung betrifft daher auch die Verwendung von Isohirudinen der Formel (A) als Blutgerinnungshemmer zur Anwendung bei der Prophylaxe und Therapie thromboembolischer Prozesse sowie deren Anwendung als Diagnostika und Reagenzien. Ferner können die erfindungsgemäßen Isohirudine auch an einen Träger gekoppelt werden, wodurch Derivate mit verzögerter thrombininhibitorischer Wirkung entstehen, wie in der deutschen Patentanmeldung P 38 19 079.6 vorgeschlagen.

Die Erfindung betrifft weiterhin pharmazeutische Zubereitungen, die ein Isohirudin der Formel (A) in einem pharmazeutisch unbedenklichen Träger enthalten.

Diese Zubereitungen können insbesondere bei den oben angegebenen Indikationen Verwendung finden, wenn sie z.B. parenteral (wie intravenös, intracutan, intramuskulär oder subcutan), oral oder topisch verabreicht werden. Die Dosierung hängt in erster Linie von der spezifischen Verabreichungsform und vom Zweck der Therapie bzw. Prophylaxe ab. Die Größe der Einzeldosen sowie das Verabreichungsschema kann am besten anhand einer individuellen Beurteilung des jeweiligen Krankheitsfalles bestimmt werden; die dazu erforderlichen Methoden zur Bestimmung von relevanten Blutfaktoren sind dem Fachmann geläufig. Im Normalfall liegt bei einer Injektion die therapeutisch wirksame Menge der erfindungsgemäßen Isohirudine im Dosisbereich von etwa 0,005 bis etwa 0,1 mg/kg Körpergewicht. Bevorzugt wird der Bereich von etwa 0,01 bis etwa 0,05 mg/kg Körpergewicht. Die Verabreichung erfolgt durch intravenöse, intramuskuläre oder sucutane Injektion. Dementsprechend enthalten pharmazeutische Präparate zur parenteralen Verabreichung in Einzeldosis-Form in Abhängigkeit von der Applikationsart pro Dosis etwa 0,4 bis etwa 7,5 mg des erfindungsgemäßen Isohirudins. Neben dem Wirkstoff enthalten diese pharmazeutischen Zubereitungen üblicherweise noch einen Puffer, z.B. einen Phosphatpuffer, der den pH-Wert zwischen etwa 3,5 und 7 halten soll, und ferner Natriumchlorid, Mannit oder Sorbit zur Einstellung der Isotonie. Sie können in gefriergetrockneter oder gelöster Form vorliegen, wobei Lösungen, mit Vorteil ein antibakteriell wirkendes Konservierungsmittel, z.B. 0,2 bis 0,3 % 4-Hydroxybenzoesäuremethylester oder -ethylester, enthalten können.

Ein Präparat für die topische Anwendung kann als wäßrige Lösung Lotion oder Gelee, ölige Lösung oder Suspension, oder fetthaltige oder insbesondere Emulsions-Salbe vorliegen. Ein Präparat in Form einer wäßrigen Lösung erhält man beispielsweise dadurch, daß man die erfindungsgemäßen Wirkstoffe oder ein therapeutisch anwendbares Salz davon in einer wäßrigen Pufferlösung von pH 4 bis 6,5 löst und gewünschtenfalls einen weiteren Wirkstoff, z.B. ein Antiinflammatorikum, und/oder ein polymeres Haftmittel, z.B. Polyvinylpyrrolidon, und/oder ein Konservierungsmittel zufügt. Die Konzentration des Wirkstoffs beträgt etwa 0,08 bis etwa 1,5 mg, vorzugsweise 0,25 bis 1,0 mg, in etwa 10 ml einer Lösung bzw. 10 g eines Gelees.

Eine ölige Applikationsform für die topische Verabreichung erhält man beispielsweise durch Suspendieren der erfindungsgemäßen Wirkstoffe oder eines therapeutisch anwendbaren Salzes davon in einem Öl, gegebenenfalls unter Zusatz von Quellmitteln, wie Aluminiumstearat, und/oder grenzflächenaktiven Mitteln (Tensiden), deren HLB-Wert ("hydrophilic-lipophilic-balance") unter 10 liegt, wie Fettsäuremonoester mehrwertiger Alkohole, z.B. Glycerinmonostearat, Sorbitanmonolaurat, Sorbitanmonostearat oder Sorbitanmonooleat. Eine fetthaltige Salbe erhält man z.B. durch Suspendieren der erfindungsgemäßen Wirkstoffe oder deren Salze in einer streichbaren Fettgrundlage, gegebenenfalls unter Zusatz eines Tensids vom HLB-Wert unter 10. Eine Emulsionssalbe erhält man durch Verreiben einer wäßrigen Lösung der erfindungsgemäßen Wirkstoffe oder deren Salze in einer weichen, streichbaren Fettunterlage unter Zusatz eines Tensids, dessen HLB-Wert unter 10 liegt. Alle diese topischen Applikationsformen können auch Konservierungsmittel enthalten. Die Konzentration des Wirkstoffes beträgt etwa 0,08 bis etwa 1,5 mg, vorzugsweise 0,25 bis 1,0 mg, in etwa 10 g der Grundmasse.

Neben den oben beschriebenen und ihnen analogen pharmazeutischen Zusammensetzungen, welche für einen direkten medizinischen Einsatz am Körper des Menschen oder eines Säugetieres bestimmt sind, betrifft die vorliegende Erfindung auch pharmazeutische Zusammensetzungen und Präparate zur medizinischen Anwendung außerhalb des lebenden Körpers des Menschen oder der Säugetiere. Solche Zusammensetzungen und Präparate verwendet man in erster Linie als gerinnungshemmenden Zusatz zu Blut, welches außerhalb des Körpers einer Zirkulation oder Behandlung (z.B. Dialyse in künstlichen Nieren), Konservierung oder Modifizierung (z.B. Haemoseparation) unterzogen wird. In ihrer Zusammensetzung sind derartige Präparate, wie Vorratslösungen oder auch Zubereitungen in Einzeldosis-Form, den oben beschriebenen Injektionspräparaten ähnlich; zweckmäßigerweise wird aber die Wirkstoffmenge bzw. -konzentration auf das Volumen des zu behandelnden Blutes oder, noch exakter, auf dessen Thrombingehalt bezogen. Dabei ist zu beachten, daß die erfindungsgemäßen Wirkstoffe (in freier Form)

(a) eine etwa 5-fache Gewichtsmenge Thrombin völlig desaktivieren;

(b) auch in größeren Mengen physiologisch harmlos sind; und

(c) vom zirkulierenden Blut auch in hohen Konzentrationen sehr schnell ausgeschieden werden, so daß keine Gefahr einer Überdosierung, auch z.B. bei Transfusionen, besteht. Je nach dem spezifischen Zweck beträgt die geeignete Dosis etwa 0,01 bis etwa 1,0 mg Wirkstoff/l Blut, wobei die obere Grenze ohne Gefahr noch weit überschritten werden darf.

Zum Gegenstand der vorliegenden Erfindung gehört auch die bioanalytische Anwendung der erfindungsgemäßen Verbindungen und ihrer Salze zur Bestimmung von Thrombin, sowie die diesem Zweck dienenden, die erfindungsgemäßen Wirkstoffe enthaltenden Präparate, z.B. Feststoffgemische und vor allem Lösungen, insbesondere wäßrige Lösungen; diese können neben einer genauen Menge bzw. Konzentration der erfindungsgemäßen Wirkstoffe (auch in Form eines Salzes) zweckmäßig noch inerte Hilfsstoffe enthalten, z.B. die oben bei Injektionspräparaten erwähnten, welche beispielsweise eine stabilisierende und/oder konservierende Aufgabe haben. Diese Präparate werden bei Bioanalysen in analoger, bekannter Weise wie die Hirudin-Präparate, beispielsweise zur Bestimmung von Thrombin, verwendet.

Darüber hinaus sind die erfindungsgemäßen Verbindungen zur Blutkonservierung brauchbar. Zu diesem Zweck setzt man die erfindungsgemäßen Verbindungen Blutkonserven vorteilhaft in einer Menge von 0,1-2 Gew.-% zu.

Die nachfolgenden Beispiele dienen zur Erläuterung der Erfindung. Hier wird im Gegensatz zu der vorhergehenden Beschreibung und den Ansprüchen der Einbuchstabencode als Kurzbezeichnung für Aminosäuren verwendet.

| Drei- und Einbuchstabencode der Aminosäuren | | | |
|---|---|---|---|
| Aminosäure | Abkürzung | Aminosäure | Abkürzung |
| Alanin | Ala (A) | Prolin | Pro (P) |
| Arginin | Arg (R) | Serin | Ser (S) |
| Cystein | Cys (C) | Threonin | Thr (T) |
| Glycin | Gly (G) | Tryptophan | Trp (W) |
| Histidin | His (H) | Tyrosin | Tyr (Y) |
| Isoleucin | Ile (I) | Valin | Val (V) |
| Leucin | Leu (L) | Asparaginsäure | Asp (D) |
| Lysin | Lys (K) | Asparagin | Asn (N) |
| Methionin | Met (M) | Glutaminsäure | Glu (E) |
| Phenylalanin | Phe (F) | Glutamin | Gln (Q) |

**Beispiel 1**

Isolierung der Isohirudine Ia, I, Ib, IIa, II, IIb, IIIa, IIIa, IIIb, Oxydation, Tryptische Spaltung, Sequenzanalyse und Aminosäureanalyse

a) Isolierung

Die Isolierung der neuen Isohirudine folgte zunächst dem aus der Literatur bekannten Verfahren (P. Walsmann, Trombosis Research 40 (1985) 563-569). Die wichtigsten Schritte sollen hier nur erwähnt werden. Nach der Extraktion des vorderen Teils der Blutegel mit 40 % Aceton und Präzipitation von Ballaststoffen mit Eisessig wurde der angereicherte Hirudinextrakt mit Aceton ausgefällt. Der Niederschlag wurde über Ionenaustausch (z.B. ®DEAE-Sephacel) in Tris/HCl-Puffer (pH 7,4) mit Hilfe eines Kochsalzgradienten gereinigt. Die im Antithrombintest aktiven Fraktionen wurden vereinigt und gefriergetrocknet. Die Pufferbestandteile wurden anschließend über ®Sephadex G 25 in Wasser portionsweise getrennt, die aktiven Fraktionen in 0,1 M Tris/HCl-Puffer (pH 8,0) auf eine Thrombin-Sepharose-Säule aufgebracht und nach dem Waschen mit demselben Puffer mit 0,1 M Natriumacetat/0,5 M Natriumchlorid (pH 5,0) eluiert. Dabei wurde auch ein Teil des Hirudins eluiert. Die reinen Hirudin-Fraktionen wurden dann mit 0,1 M Tris/HCl/1,5 M Benzamidin (pH 8,0) eluiert. Durch Kopplung der Thrombin-Sepharose auf eine ®Sephadex G 25-Säule wurde anschließend bei reduziertem Fluß die Elution des Hirudins mit einer Trennung des Thrombin-Inhibitors Benzamidin zusammen vorgenommen. Nach Gefriertrocknung wurden die aktiven Fraktionen durch Ionenaustauschchromatographie über SE-®Sephadex in 0,01 M Ammoniumacetat-Puffer (pH 3,8) und Elution mit einem Gradienten von Natriumchlorid gereinigt. Es wurden drei aktive Fraktionen isoliert. Die Fraktion II wurde über ®Sephadex entsalzt und gefriergetrocknet. Die spezifische Aktivität liegt bei 12 AT-U/µg.

Die nicht einheitliche Fraktion II wurde für die Micro-Bore RP-HPLC eingesetzt. Unter Verwendung von sehr flachen Gradienten (0,06 B % pro Minute) wurde das Gemisch in drei Hauptpeaks mit sehr unterschiedlichen Retentionszeiten (RT) gelöst (Peak I: RT ca. 89 Minuten: Peak II: RT ca. 113 Minuten; Peak III: RT ca. 134 Minuten; siehe Figur 1). Diese Hauptpeaks sind von kleineren Satellitenpeaks (a und b) begleitet. Peak IIIa bezeichnet z.B. den Satellitenpeak mit der RT ca. 130 Minuten, der unmittelbar vor dem Hauptpeak III eluiert wird. Entsprechend wird der Satellitenpeak IIIb (RT ca. 139 Minuten) verwendet, der nach dem Hauptpeak III eluiert wird.

Durch vorsichtige Erhöhung der eingespritzten Menge und Sammlung der Fraktionen (Peaks) wurde die Micro-Bore RP-HPLC-Anlage zur präparativen Isolierung der verschiedenen Isohirudin-Varianten verwendet.

10

Bis zu 50 μg wurden eingespritzt und die verschiedenen Fraktionen (Peaks) gesammelt, ohne Einbußen in der Auflösung der Peaks zu bemerken. Um eine ausreichende Menge zu isolieren, wurde dieser Vorgang mehrere Male wiederhalt und die identischen Fraktionen vereinigt und gefriergetrocknet. Anschließend wurden die verschiedenen Peak-Rechromatographien durch "engere" Fraktionierungen, endgereinigt. Auf diese Weise wurden die Isohirudine Ia, I, Ib, IIa, II, IIb, IIIa, III und IIIb getrennt.

Die reinen Substanzen wurden nach Bedarf oxidiert und mit Trypsin gespalten.

b) Oxydative Spaltung der Disulfide

Die Isohirudine wurden mit 100 μl Perameisensäurelösung versetzt (hergestellt aus 50 μl Wasserstoffperoxid und 950 μl Ameisensäure; 2,5 Stunden bei Raumtemperatur stehen gelassen). Nach 30 Minuten wurde mit 200 μl Wasser verdünnt und anschließend gefriergetrocknet.

c) Trypsin Spaltung

Die oxydierten Isohirudine wurden in 50 μl NMM-Puffer (0,2 M N-Methylmorpholin mit Eisessig eingestellt auf pH 8) aufgenommen. Dazu wurde eine Lösung von Trypsin in NMM-Puffer im Verhältnis Substrat/Enzym = 1:100 zugegeben. Nach 30 Minuten bei Raumtemperatur wurde die Reaktion durch Zugabe von 100 μl Eisessig gestoppt. Dieses erhaltene Gemisch wurde dann weiter für die Micro-Bore RP-HPLC verwendet.

d) Sequenzanalyse

Die isolierten und gereinigten Isohirudine wurden mit einem 477A Pulsed Liquid Phase (TM) Protein Sequenzer (Fa. Applied Biosystems) sequenziert, der mit einem 120A PTH-Analysator mit Datenauswertsystem on-line gekoppelt ist. Es wurde nach den Angaben des Herstellers gearbeitet. Die Ergebnisse sind in den Tabellen 3 bis 15 dargestellt.

e) Aminosäureanalyse

Die Aminosäurezusammensetzung der einzelnen Isohirudine wurde mit einem Aminosäureanalysator Beckman 6300 ermittelt. Dabei wurde nach Anleitung des Herstellers gearbeitet. Die Reagenzien und die Trennsäule wurden ebenfalls vom Hersteller bezogen. Die anschließende Integration erfolgte mit dem LAS-System (Hewlett Packard). Die Proteine (ca. 30-50 pmol) wurden in einem 4 x 40 mm Quartzreagenzglas getrocknet und in der Dampfphase von azeotropischem HCl/0,8 % Phenol unter Stickstoffatomosphäre bei 110 °C hydrolysiert. Für jeden Ansatz wurden 500 pmol Insulin-Standard mitanalysiert. Die nachfolgende Tabelle 2 zeigt die ermittelten Ergebnisse.

## Tabelle 2: Aminosäureanalyse

| Isohirudine | Ia a | Ia b | I a | I b | IIa a | IIa b | II a | II b | IIb a | IIb b |
|---|---|---|---|---|---|---|---|---|---|---|
| Asx | 9,6 | 9 | 9,8 | 10 | 9,9 | 10 | 9,1 | 9 | 9,2 | 9 |
| Thr | 3,8 | 4 | 3,7 | 4 | 4,7 | 5 | 4,5 | 5 | 4,6 | 5 |
| Ser | 3,4 | 4 | 3,4 | 4 | 3,6 | 4 | 5,6 | 4 | 3,7 | 4 |
| Glx | 12,2 | 12 | 13,3 | 12 | 13,2 | 13 | 13,9 | 13 | 13,2 | 13 |
| Pro | 2,3 | 3 | 2 | 3 | 2,2 | 3 | 2,1 | 3 | 2,1 | 3 |
| Gly | 9,3 | 9 | 9 | 9 | 9,6 | 9 | 10,1 | 9 | 9,1 | 9 |
| Cys/2 | 4 | 6 | 4,5 | 6 | 3,9 | 6 | 3,6 | 6 | 4,5 | 6 |
| Val | 2,9 | 4 | 3,0 | 4 | 1,9 | 2 | 1,9 | 2 | 1,8 | 2 |
| Ile | 1,8 | 2 | 1,8 | 2 | 1,7 | 2 | 2,3 | 2 | 1,8 | 2 |
| Leu | 3,7 | 4 | 4,0 | 4 | 3,8 | 4 | 3,4 | 4 | 4 | 4 |
| Tyr | 1,9 | 2 | 1,9 | 2 | 1,8 | 2 | 0,6 | 2 | 1,9 | 2 |
| Phe | 0,9 | 1 | 0,9 | 1 | 1 | 1 | 0,9 | 1 | 0,9 | 1 |
| His | 1 | 1 | 0,9 | 1 | 0,9 | 1 | 1,3 | 1 | 0,1 | 1 |
| Lys | 2,8 | 4 | 2,9 | 3 | 3,1 | 3 | 2,5 | 3 | 2,8 | 3 |

EP 0 364 942 B1

| Isohirudine | IIIa | | III | | IIIb | |
|---|---|---|---|---|---|---|
| | a | b | a | b | a | b |
| Asx | 10 | 10 | 10 | 10 | 9,5 | 10 |
| Thr | 3,9 | 4 | 3,8 | 4 | 3,7 | 4 |
| Ser | 3,7 | 4 | 4 | 4 | 3,2 | 4 |
| Glx | 13,6 | 13 | 13,7 | 13 | 13,1 | 13 |
| Pro | 2,1 | 3 | 2,2 | 3 | 2,4 | 3 |
| Gly | 9,4 | 9 | 9,4 | 9 | 9,1 | 9 |
| Cys/2 | 4,8 | 6 | 3,9 | 6 | 4,1 | 6 |
| Val | 2,9 | 4 | 2,5 | 4 | 2,8 | 4 |
| Ile | 1,9 | 2 | 1,7 | 2 | 1,9 | 2 |
| Leu | 4,2 | 4 | 3,9 | 4 | 4 | 4 |
| Tyr | 1,7 | 2 | 1,2 | 2 | 1,7 | 2 |
| Phe | 1 | 1 | 1 | 1 | 1 | 1 |
| His | 0,9 | 1 | 1 | 1 | 0,9 | 1 |
| Lys | 3 | 3 | 2,9 | 3 | 2,7 | 3 |

a: gefunden

b: berechnet

In den nachfolgenden Beispielen 2 bis 9 wurde analog den in Beispiel 1 angegebenen Verfahren gearbeitet. Die entsprechenden Angaben über die spezifische Aktivität und die Aminosäureanalysen sind in den Tabellen 1 und 2 angegeben.

**Beispiel 2**

Isohirudin I

Das Isohirudin I wurde aus dem Peak I (RT ca. 89 Minuten) isoliert und durch Rechromatographie gereinigt. Die N-terminale Sequenzanalyse ermöglichte die Struktur bis zu 30 Aminosäuren zu bestimmen (Tabelle 3). Nach Oxydation und tryptischer Spaltung wurden die Fragmente T2 und T3 (C-terminales Peptid) isoliert und die Sequenzen aufgeklärt. Die Ergebnisse sind in Tabelle 4 dargestellt.

13

**Tabelle 3**

Quantitative Sequenzanalyse des Isohirudins I

| | 1 | | | | 5 | | | | | 10 | | |
|---|---|---|---|---|---|---|---|---|---|---|---|---|
| Aminosäure | V | V | Y | T | D | C | T | E | S | G | Q | N |
| pmol | 107 | 90 | 76 | 48 | 30 | nb | 21 | 29 | 13 | 30 | 19 | 20 |

| | | | 15 | | | | | 20 | | | | |
|---|---|---|---|---|---|---|---|---|---|---|---|---|
| Aminosäure | L | C | L | C | E | G | S | N | V | C | G | N* |
| pmol | 18 | nb | 6 | nb | 11 | 16 | 12 | 17 | 15 | nb | 12 | 13 |

| | 25 | | | | | 30 | | | | | 35 | |
|---|---|---|---|---|---|---|---|---|---|---|---|---|
| Aminosäure | G | N | K | C | I | L | G | S | D | G | E | K |
| pmol | 11 | 8 | 6 | nb | 5 | 5 | | | | | | |

| | | | | 40 | | | | | | 45 | | |
|---|---|---|---|---|---|---|---|---|---|---|---|---|
| Aminosäure | N | Q | C | V | T | G | E | G | T | P | K | P |
| pmol | | | | | | | | | | | | |

| | | 50 | | | | | 55 | | | | | 60 |
|---|---|---|---|---|---|---|---|---|---|---|---|---|
| Aminosäure | Q | S | H | N | D | G | D | F | E | E | I | P |
| pmol | | | | | | | | | | | | |

| | | | | 65 | |
|---|---|---|---|---|---|
| Aminosäure | E | E | Y | L | Q |
| pmol | | | | | |

* Aminosäureaustausch im Vergleich zu (1)

nb: nicht bestimmbar

V V Y T D C T E S G Q N L C L C E G S N
V C G Q G N K C I L G S D G E K N Q C V
T G E G T P K P Q S H N D G D F E E I P
E E Y* L Q

(1)

Y* Sulfatotyrosin

**Tabelle 4**

Quantitative Sequenzanalyse der tryptischen Spaltprodukte
des Isohirudins I

14

Peptid T2

| Aminosäure T2 | 28 C | I | 30 L | G | S | D | G | 35 E | K |
|---|---|---|---|---|---|---|---|---|---|
| pmol | nb | 204 | 156 | 128 | 40 | 63 | 68 | 23 | 8 |

Peptid T3

| Aminosäure T3 | 37 N | Q | C | 40 V | T | G | E | G | 45 T | P | K | P |
|---|---|---|---|---|---|---|---|---|---|---|---|---|
| pmol | 159 | 178 | nb | 105 | 89 | 122 | 89 | 86 | 63 | 57 | 58 | 25 |

| Aminosäure T3 | Q | 50 S | H | N | D | G | 55 D | F | E | E | I | 60 P |
|---|---|---|---|---|---|---|---|---|---|---|---|---|
| pmol | 36 | 12 | 6 | 12 | 9 | 10 | 11 | 7 | 7 | 3 | 4 | 5 |

| Aminosäure T3 | E | E | Y | L | 65 Q |
|---|---|---|---|---|---|
| pmol | 4 | 4 | 1 | 1 | 3 |

nb: nicht bestimmbar

**Beispiel 3**

Isohirudin Ia

Analog Beispiel I wurde Isohirudin Ia aus dem Proteingemisch, das vor dem Peak I erhalten wurde, isoliert (RT ca. 85 Minuten). Das Produkt wurde durch Rechromatographie gereinigt. Die anschließende N-terminale Sequenzanalyse ermöglichte, die gesamte Struktur zu ermitteln (Tabelle 5).

## Tabelle 5

Quantitative Sequenzanalyse des Isohirudins Ia

| Aminosäure | 1 V | V | Y | T | 5 D | C | T | E | S | 10 G | Q | N |
|---|---|---|---|---|---|---|---|---|---|---|---|---|
| pmol | 336 | 334 | 297 | 614 | 161 | nb | 339 | 114 | 241 | 185 | 196 | 181 |

|  |  |  | 15 |  |  |  |  | 20 |  |  |  |  |
|---|---|---|---|---|---|---|---|---|---|---|---|---|
| Aminosäure | L | C | L | C | E | G | S | N | V | C | G | K* |
| pmol | 188 | nb | 173 | nb | 123 | 156 | 130 | 149 | 146 | nb | 132 | 118 |

|  | 25 |  |  |  |  | 30 |  |  |  | 35 |  |  |
|---|---|---|---|---|---|---|---|---|---|---|---|---|
| Aminosäure | G | N | K | C | I | L | G | S | D | G | E | K |
| pmol | 122 | 118 | 78 | nb | 71 | 84 | 71 | 53 | 69 | 76 | 60 | 69 |

|  |  |  | 40 |  |  |  | 45 |  |  |  |  |
|---|---|---|---|---|---|---|---|---|---|---|---|
| Aminosäure | N | Q | C | V | T | G | E | G | T | P | K | P |
| pmol | 60 | 61 | nb | 46 | 39 | 43 | 37 | 39 | 24 | 16 | 28 | 19 |

|  | 50 |  |  |  |  | 55 |  |  |  | 60 |
|---|---|---|---|---|---|---|---|---|---|---|
| Aminosäure | Q | S | H | N | D | G | D | F | E | E | I | P |
| pmol | 17 | 11 | 6 | 16 | 12 | 9 | 11 | 13 | 6 | 5 | 5 | 3 |

|  |  |  |  | 65 |
|---|---|---|---|---|
| Aminosäure | E | E | Y | L | Q |
| pmol | 4 | 4 | 3 | 3 | 2 |

\* Aminosäureaustausch im Vergleich zu (1)

nb: nicht bestimmbar

**Beispiel 4**

Isohirudin IIa

Das Isohirudin IIa wurde aus dem Peak IIa (RT ca. 107 Minuten) isoliert und durch Rechromatographie gereinigt. Durch N-terminale Sequenzanalyse wurde die Struktur aufgeklärt (Tabelle 6).

## Tabelle 6

## Quantitative Sequenzanalyse des Isohirudins IIa

|  | 1 |  |  |  | 5 |  |  |  |  | 10 |  |  |
|---|---|---|---|---|---|---|---|---|---|---|---|---|
| Aminosäure | I* | T* | Y | T | D | C | T | E | S | G | Q | N |
| pmol | 423 | 372 | 377 | 310 | 368 | nb | 392 | 274 | 295 | 261 | 287 | 271 |

16

| | | 15 | | | | | 20 | | | | |
|---|---|---|---|---|---|---|---|---|---|---|---|
| Aminosäure | L | C | L | C | E | G | S | N | V | C | G | N* |
| pmol | 258 | nb | 241 | nb | 230 | 228 | 227 | 226 | 218 | nb | 223 | 210 |

| 25 | | | | | 30 | | | | | 35 | |
|---|---|---|---|---|---|---|---|---|---|---|---|
| Aminosäure | G | N | K | C | K* | L | G | S | D | G | E | E* |
| pmol | 191 | 149 | 200 | nb | 171 | 182 | 169 | 176 | 162 | 145 | 135 | 125 |

| | | | 40 | | | | | 45 | | | |
|---|---|---|---|---|---|---|---|---|---|---|---|
| Aminosäure | N | Q | C | V | T | G | E | G | T | P | K | P |
| pmol | 115 | 126 | nb | 107 | 105 | 96 | 93 | 92 | 79 | 72 | 71 | 62 |

| | 50 | | | | | 55 | | | | 60 | |
|---|---|---|---|---|---|---|---|---|---|---|---|
| Aminosäure | Q | S | H | N | D | G | D | F | E | E | I | P |
| pmol | 60 | 49 | 55 | 52 | 50 | 41 | 43 | 47 | 30 | 31 | 30 | 16 |

| | | | | 65 |
|---|---|---|---|---|
| Aminosäure | E | E | Y | L | Q |
| pmol | 15 | 15 | 17 | 12 | 12 |

**\* Aminosäureaustusch im Vergleich zu (1)**

**nb: nicht bestimmbar**

**Beispiel 5**

Isohirudine II und II′

Analog Beispiel I wurden die Isohirudine II und II′ im Gemisch aus der Fraktion II (Peak II; RT ca. 113 Minuten) isoliert und durch Rechromatographie gereinigt. Durch N-terminale Sequenzanalyse wurde die Sequenz der Isohirudine bis zur Aminosäure 35 aufgeklärt. Das C-terminale Peptid (28-65) wurde nach Oxydation und tryptischer Spaltung isoliert und die Sequenz ermittelt (Tabelle 8). Die Gesamtsequenzanalyse der Isohirudine II und II′ ist in Tabelle 7 angegeben. Das Isohirudin II′ wurde dabei wie bereits vorher erläutert, während der Sequenzanalyse des Isohirudin-Gemisches II und II′ charakterisiert. Isohirudin II′ unterscheidet sich von dem Isohirudin II durch den Austausch von Asparagin durch Asparaginsäure in der Position 20.

## Tabelle 7

Quantitative Sequenzanalyse der Isohirudine II und II'

| | 1 | | | | 5 | | | | | 10 | | |
|---|---|---|---|---|---|---|---|---|---|---|---|---|
| Aminosäure II | I* | T* | Y | T | D | C | T | E | S | G | Q | D* |
| pmol | 311 | 268 | 278 | 267 | 242 | nb | 169 | 175 | 165 | 144 | 165 | 163 |

| | | | 15 | | | | | 20 | | | | |
|---|---|---|---|---|---|---|---|---|---|---|---|---|
| Aminosäure II | L | C | L | C | E | G | S | N | V | C | G | K* |
| pmol | 150 | nb | 132 | nb | 124 | 109 | 116 | 124 | 110 | nb | 111 | 110 |
| Aminosäure II' | | | | | | | | D** | | | | |
| pmol | | | | | | | | 4 | | | | |

| | 25 | | | | | 30 | | | | | 35 | |
|---|---|---|---|---|---|---|---|---|---|---|---|---|
| Aminosäure II | G | N | K | C | I | L | G | S | N* | G | E | E* |
| pmol | 98 | 89 | 74 | nb | 66 | 55 | 43 | 70 | 49 | 51 | 45 | 28 |

| | | | | 40 | | | | | 45 | | | |
|---|---|---|---|---|---|---|---|---|---|---|---|---|
| Aminosäure II | N | Q | C | V | T | G | E | G | T | P | K | P |
| pmol | 20 | 22 | nb | 17 | 12 | 13 | 10 | 3 | 15 | | | |

| | | 50 | | | | | 55 | | | | | 60 |
|---|---|---|---|---|---|---|---|---|---|---|---|---|
| Aminosäure II | Q | S | H | N | D | G | D | F | E | E | I | P |
| pmol | | | | | | | | | | | | |

| | | | | | 65 |
|---|---|---|---|---|---|
| Aminosäure II | E | E | Y | L | Q |
| pmol | | | | | |

\* Aminosäureaustausch im Vergleich zu (1)

** Aminosäureaustausch im Vergleich zu II

nb: nicht bestimmbar

## Tabelle 8

Quantitative Sequenzanalyse der tryptischen Spaltprodukte der Isohirudine II und II'

| | 28 | | 30 | | | | | 35 | | | | |
|---|---|---|---|---|---|---|---|---|---|---|---|---|
| Aminosäure | C | I | L | G | S | N* | G | E | E* | N | Q | C |
| pmol | nb | 266 | 242 | 250 | 223 | 217 | 184 | 178 | 178 | 169 | 145 | nb |

| | 40 | | | | | 45 | | | | 50 | | |
|---|---|---|---|---|---|---|---|---|---|---|---|---|
| Aminosäure | V | T | G | E | G | T | P | K | P | Q | S | H |
| pmol | 144 | 125 | 138 | 126 | 125 | 137 | 118 | 112 | 102 | 125 | 91 | 113 |

| | | | 55 | | | | | 60 | | | | |
|---|---|---|---|---|---|---|---|---|---|---|---|---|
| Aminosäure | N | D | G | D | F | E | E | I | P | E | E | Y |
| pmol | 117 | 100 | 106 | 97 | 99 | 83 | 79 | 80 | 68 | 59 | 63 | 41 |

| | | 65 |
|---|---|---|
| Aminosäure | L | Q |
| pmol | 38 | 16 |

* Aminosäureaustausch im Vergleich zu (1)

nb: nicht bestimmbar

### Beispiel 6

Isohirudin IIb

Analog Beispiel 1 wurde Isohirudin IIb aus dem Proteingemisch nach Peak II (RT ca. 115 Minuten) isoliert und durch Rechromatographie gereinigt. Durch N-terminale Sequenzanalyse wurde die gesamte, in Tabelle 9 dargestellte Sequenz ermittelt. Zusätzlich wurde noch eine Trypsinspaltung durchgeführt, die zur Isolierung des Peptids 28-65 (Tabelle 10) führte. Die entsprechende Sequenz wurde ermittelt.

## Tabelle 9

Quantitative Sequenzanalyse des Isohirudins IIb

| Aminosäure | 1 I* | T* | Y | T | 5 D | C | T | E | S | 10 G | Q | N |
|---|---|---|---|---|---|---|---|---|---|---|---|---|
| pmol | 378 | 502 | 244 | 465 | 445 | nb | 377 | 400 | 396 | 303 | 291 | 281 |

| Aminosäure | L | C | 15 L | C | E | G | S | N | 20 V | C | G | K* |
|---|---|---|---|---|---|---|---|---|---|---|---|---|
| pmol | 279 | nb | 269 | nb | 240 | 248 | 248 | 235 | 235 | nb | 259 | 231 |

| Aminosäure | 25 G | N | K | C | I | 30 L | G | S | N* | G | 35 E | E* |
|---|---|---|---|---|---|---|---|---|---|---|---|---|
| pmol | 215 | 230 | 219 | nb | 221 | 222 | 217 | 210 | 206 | 200 | 195 | 195 |

| Aminosäure | N | Q | C | 40 V | T | G | E | G | 45 T | P | K | P |
|---|---|---|---|---|---|---|---|---|---|---|---|---|
| pmol | 185 | 174 | nb | 163 | 156 | 145 | 132 | 140 | 129 | 112 | 105 | 91 |

| Aminosäure | Q | 50 S | H | N | D | G | 55 D | F | E | E | I | 60 P |
|---|---|---|---|---|---|---|---|---|---|---|---|---|
| pmol | 86 | 80 | 72 | 71 | 65 | 54 | 51 | 42 | 46 | 33 | 24 | 26 |

| Aminosäure | E | E | Y | L | 65 Q |
|---|---|---|---|---|---|
| pmol | 18 | 18 | 15 | 12 | 10 |

* Aminosäureaustausch im Vergleich zu (1)

## Tabelle 10

Quantitative Sequenzanalyse der tryptischen Peptide des Isohirudins IIb

| Aminosäure | 28 C | I | 30 L | G | S | N* | G | E | 35 E* | N | Q | C |
|---|---|---|---|---|---|---|---|---|---|---|---|---|
| pmol | 300 | 379 | 381 | 368 | 347 | 337 | 332 | 316 | 305 | 293 | 271 | nb |

| Aminosäure | 40 V | T | G | E | G | T | 45 P | K | P | Q | 50 S | H |
|---|---|---|---|---|---|---|---|---|---|---|---|---|
| pmol | 255 | 249 | 234 | 228 | 212 | 205 | 197 | 167 | 154 | 143 | 133 | 127 |

| Aminosäure | N | D | G | D (55) | F | E | E | I | P (60) | E | E | Y |
|---|---|---|---|---|---|---|---|---|---|---|---|---|
| pmol | 112 | 102 | 115 | 100 | 91 | 81 | 78 | 64 | 54 | 32 | 22 | 18 |

| Aminosäure | L | Q (65) |
|---|---|---|
| pmol | 14 | 12 |

* Aminosäureaustausch im Vergleich zu (1)

nb: nicht bestimmbar

**Beispiel 7**

Isohirudine IIIa und IIIa′

Die Isohirudine IIIa und IIIa′ wurden aus dem Peak mit der RT ca. 126 Minuten isoliert und durch Rechromatographie gereinigt. Die N-terminale Sequenzanalyse ermöglichte die Struktur bis zum Carboxy-Terminus zu bestimmen (Tabelle 11). Das Isohirudin IIIa′ wurde wie bereits vorher erläutert, während der Sequenzanalyse charakterisiert. Das Isohirudin IIIa′ unterscheidet sich von dem Isohirudin IIIa durch Austausch von Glutaminsäure gegen Glutamin in der Position 11, Asparaginsäure gegen Asparagin in der Position 12, Glycin gegen Asparaginsäure in der Positon 18 und Asparaginsäure gegen Asparagin in der Position 33.

**Tabelle 11**

**Quantitative Sequenzanalyse der Isohirudine IIIa und IIIa'**

| Aminosäure IIIa | V (1) | V | Y | T | D (5) | C | T | E | S | G (10) | Q | N |
|---|---|---|---|---|---|---|---|---|---|---|---|---|
| pmol | 660 | 675 | 666 | 477 | 407 | nb | 321 | 458 | 238 | 390 | 269 | 282 |

| Aminosäure IIIa' | | | | | | | | | | | E** | D** |
|---|---|---|---|---|---|---|---|---|---|---|---|---|
| pmol | | | | | | | | | | | 46 | 33 |

| | | | 15 | | | | | 20 | | | | |
|---|---|---|---|---|---|---|---|---|---|---|---|---|
| Aminosäure IIIa | L | C | L | C | E | D* | S | N | V | C | G | E* |
| pmol | 318 | nb | 284 | nb | 269 | 247 | 239 | 227 | 218 | nb | 204 | 185 |

| | | | | | | G** | | | | | | E* |
|---|---|---|---|---|---|---|---|---|---|---|---|---|
| Aminosäure IIIa' | | | | | | | | | | | | |
| pmol | | | | | | 11 | | | | | | |

| | 25 | | | | | 30 | | | | | 35 | |
|---|---|---|---|---|---|---|---|---|---|---|---|---|
| Aminosäure IIIa | G | N | K | C | I | L | G | S | N* | G | E | K |
| pmol | 178 | 169 | 161 | nb | 150 | 157 | 154 | 137 | 131 | 128 | 125 | 120 |

| | | | | | | | | | D** | | | |
|---|---|---|---|---|---|---|---|---|---|---|---|---|
| Aminosäure IIIa' | | | | | | | | | | | | |
| pmol | | | | | | | | | 4 | | | |

| | | | | 40 | | | | | 45 | | | |
|---|---|---|---|---|---|---|---|---|---|---|---|---|
| Aminosäure IIIa | N | E* | C | V | T | G | E | G | T | P | K | P |
| pmol | 88 | 84 | nb | 73 | 62 | 69 | 63 | 59 | 51 | 45 | 53 | 38 |

| | | 50 | | | | | 55 | | | | | 60 |
|---|---|---|---|---|---|---|---|---|---|---|---|---|
| Aminosäure IIIa | Q | S | H | N | D | G | D | F | E | E | I | P |
| pmol | 32 | 33 | 21 | 22 | 18 | 15 | 13 | 10 | 10 | 11 | 8 | 6 |

| | | | | | 65 | | | | | | | |
|---|---|---|---|---|---|---|---|---|---|---|---|---|
| Aminosäure IIIa | E | E | Y | L | Q | | | | | | | |
| pmol | 6 | 5 | 5 | 4 | 3 | | | | | | | |

\* Aminosäureaustausch im Vergleich zu (1)

\*\* Aminosäureaustausch im Vergleich IIIa

nb: nicht bestimmbar

**Beispiel 8**

Isohirudin III

Das Isohirudin III wurde aus dem Peak mit der RT ca. 139 Minuten isoliert und rechromatographisch gereinigt. Die N-terminale Sequenzanalyse in Kombination mit der Sequenzanalyse der tryptischen Spaltprodukte (Peptid 28-65) ermöglichte die Strukturbestimmung des gesamten Proteins (Tabellen 12 und 13). Verglichen mit allen hier beschriebenen Isohirudinen wurde das Isohirudin III in größerer Menge isoliert, so daß mit dieser Substanz zusätzlich der proteinchemische Strukturbeweis der Mutation Asparaginsäure zu Asparagin durchgeführt wurde.

Bornstein (Methods in Enzymol. 47 (1977) 132-145) beschreibt eine selektive Spaltung von Asn-Gly-Peptidbindungen mit dem nucleophilen Agenz Hydroxylamin.

Dementsprechend wurde zunächst ein Puffer hergestellt, der 6 M Guanidinium Hydrochlorid und 2 M Hydroxylamin enthält. Dazu wurden 23 g Guanidinium Hydrochlorid und 5,5 g Hydroxylamin im Eisbad mit etwas Wasser zusammengegeben und unter starkem Rühren mit 4,5 M LiOH in Lösung gebracht, wobei der pH-Wert 6,5 nicht überschritt. Anschließend wurde der pH-Wert auf 9 eingestellt und mit Wasser auf 50 ml aufgefüllt.

Das Isohirudin III (ca. 2 nmol) wurde lyophilisiert, mit 200 μl des hergestellten Puffers aufgenommen und anschließend 4 Stunden bei 45 °C im Wasserbad inkubiert. Zur Beendigung der Reaktion wurde 200 μl Eisessig (pH 3) zugegeben und die Probe eingefroren. Danach wurde die Probe auf 200 μl eingeengt und auf einer reversed-phase Säule (4,6 mm x 250 mm ), gefüllt mit C-18 modifiziertem Kieselgel, entsalzt. Der Gradient lief dabei von 0 % bis 100 % B in 30 Minuten bei einem Fluß von 1 ml/min (Puffer A: 100 % Wasser, 0,1 % TFA; Puffer B: 90 % AcCN, 10 % Wasser, 0,1 % TFA). Die erhaltene Hauptfraktion wurde anschließend mit Hilfe der Micro-Bore HPLC rechromatographiert. Es wurde eine Fraktion F1 isoliert, die nach der Charakterisierung durch Sequenzanalyse zwei N-Termina aufwies (Tabelle 14). nämlich Val Val Tyr und Gly Glu Lys.

Durch die Cysteinverknüpfung (Cys6-Cys14, Cys16-Cys28, Cys 22-Cys39) wird auch nach der Hydroxylspaltung das Isohirudin III noch zusammengehalten.

## Tabelle 12

Quantitative Sequenzanalyse des Isohirudins III

| Aminosäure III | 1 V | V | Y | T | 5 D | C | T | E | S | 10 G | Q | N |
|---|---|---|---|---|---|---|---|---|---|---|---|---|
| pmol | 615 | 569 | 468 | 377 | 265 | nb | 178 | 250 | 108 | 214 | 147 | 195 |
| Aminosäure III | L | C | 15 L | C | E | D* | S | 20 N | V | C | G | Q |
| pmol | 158 | nb | 83 | nb | 102 | 88 | 72 | 64 | 54 | nb | 50 | 48 |
| Aminosäure III | 25 G | N | K | C | I | 30 L | G | S | N* | G | 35 E | K |
| pmol | 32 | 37 | 29 | nb | 28 | 30 | 23 | 19 | 14 | 17 | 16 | 12 |
| Aminosäure III | N | Q | C | 40 V | T | G | E | G | 45 T | P | K | P |
| pmol | 10 | 9 | nb | 5 | 3 | 5 | 6 | 4 | 2 | 1 | 1 | 3 |
| Aminosäure III | Q | 50 S | H | N | D | G | 55 D | F | E | E | I | 60 P |
| pmol | 1 | 0,5 | 1 | 1 | 5 | 1,5 | 0,3 | 0,8 | 2 | 2 | 1,5 | 1 |
| Aminosäure III | E | E | Y | L | 65 Q | | | | | | | |
| pmol | 1 | 0,4 | 0,6 | 0,8 | 0,6 | | | | | | | |

* Aminosäureaustausch im Vergleich zu (1)

nb: nicht bestimmbar

## Tabelle 13

Quantitative Sequenzanalyse der tryptischen Peptide des Isohirudins III

| Aminosäure | 28 C | I | 30 L | G | S | N* | G | 35 E | K | N | Q | C |
|---|---|---|---|---|---|---|---|---|---|---|---|---|
| pmol | nb | 301 | 295 | 286 | 234 | 273 | 246 | 242 | 219 | 194 | 139 | nb |
| Aminosäure | 40 V | T | G | E | 45 G | T | P | K | P | Q | 50 S | H |
| pmol | 151 | 136 | 141 | 149 | 117 | 99 | 82 | 87 | 71 | 68 | 64 | 58 |
| Aminosäure | N | D | 55 G | D | F | E | E | I | 60 P | E | E | Y |
| pmol | 51 | 41 | 36 | 30 | 25 | 24 | 27 | 21 | 12 | 16 | 15 | 12 |
| Aminosäure | L | 65 Q | | | | | | | | | | |
| pmol | 0,7 | 2 | | | | | | | | | | |

* Aminosäureaustausch im Vergleich zu (1)

nb: nicht bestimmbar

## Tabelle 14

Quantitative Sequenzanalyse der Fraktion F1 aus der Hydroxylaminspaltung

| Abbaustufe | 1 | 2 | 3 | 4 |
|---|---|---|---|---|
| Sequenzposition | 1 | 2 | 3 | 4 |
| Aminosäure: N-terminal | V | V | Y | T |
| pmol | 17 | 17 | 24 | 22 |
| Sequenzposition | 34 | 35 | 36 | 37 |
| Aminosäure: Spaltstelle | G | E | K | N |
| pmol | 41 | 50 | 37 | 34 |

Das Auffinden von zwei N-Termina für F1 ist der eindeutige Beweis der Hydroxylaminspaltung in der Sequenz des Isohirudins III an der Peptidbindung N-G

**Beispiel 9**

Isohirudine IIIb und IIIb′

Die Isohirudine IIIb und IIIb′ wurden analog Beispiel 1 aus dem Peak mit der RT ca. 134 Minuten isoliert und durch Rechromatographie gereinigt. Durch N-terminale Sequenzanalyse wurde die Struktur des Isohirudins IIIb bis zum Carboxylende charakterisiert (Tabelle 15). Auch die Struktur des Isohirudins IIIb′, die ebenfalls bei der Sequenzanalyse ermittelt wurde, ist in Tabelle 15 dargestellt.

## Tabelle 15

### Quantitative Sequenzanalyse der Isohirudine IIIb und IIIb'

|  | 1 |  |  |  | 5 |  |  |  |  | 10 |  |  |
|---|---|---|---|---|---|---|---|---|---|---|---|---|
| Aminosäure IIIb | V | V | Y | T | D | C | T | E | S | G | Q | N |
| pmol | 530 | 511 | 499 | 433 | 460 | nb | 395 | 332 | 339 | 326 | 321 | 278 |

|  |  |  | 15 |  |  | 20 |  |  |  |  |  |  |
|---|---|---|---|---|---|---|---|---|---|---|---|---|
| Aminosäure IIIb | L | C | L | C | Q | D* | S | N | V | C | G | Q |
| pmol | 264 | nb | 258 | nb | 246 | 240 | 221 | 224 | 195 | nb | 185 | 175 |
| Aminosäure IIIb' |  |  |  |  |  | G** |  |  |  |  |  |  |
| pmol |  |  |  |  |  | 56 |  |  |  |  |  |  |

|  | 25 |  |  |  | 30 |  |  |  |  | 35 |  |  |
|---|---|---|---|---|---|---|---|---|---|---|---|---|
| Aminosäure IIIb | G | N | K | C | I | L | G | S | N* | G | E | K |
| pmol | 168 | 154 | 154 | nb | 131 | 134 | 128 | 126 | 118 | 106 | 102 | 106 |

|  |  |  | 40 |  |  |  |  |  | 45 |  |  |  |
|---|---|---|---|---|---|---|---|---|---|---|---|---|
| Aminosäure IIIb | N | Q | C | V | T | G | E | G | T | P | K | P |
| pmol | 106 | 95 | nb | 87 | 82 | 80 | 74 | 75 | 71 | 61 | 55 | 52 |

| Aminosäure IIb | | 50 | | | | | | 55 | | | | | 60 |
|---|---|---|---|---|---|---|---|---|---|---|---|---|---|
| | Q | S | H | N | D | G | D | F | E | E | I | P |
| pmol | 48 | 12 | 36 | 31 | 30 | 24 | 21 | 23 | 18 | 16 | 18 | 12 |

| Aminosäure IIb | | | | 65 | |
|---|---|---|---|---|---|
| | E | E | Y | L | Q |
| pmol | 11 | 11 | 8 | 5 | 3 |

\* Aminosäureaustausch im Vergleich zu (1)

\*\* Aminosäureaustausch im Vergleich zu IIb

nb: nicht bestimmbar

**Patentansprüche**
**Patentansprüche für folgende Vertragsstaaten : AT, BE, CH, DE, FR, GB, IT, LI, LU, NL, SE**

1.  Isohirudin der Formel (A)

```
1              5                    10
A - B - Tyr - Thr - Asp - Cys - Thr - Glu - Ser - Gly -
              15                    20
C - E - Leu - Cys - Leu - Cys - F - G - Ser - I - Val -
              25                    30
Cys - Gly - J - Gly - Asn - Lys - Cys - K - Leu - Gly -
              35                    40
Ser - L - Gly - Glu - M - Asn - N - Cys - Val - Thr - Gly -
              45                    50
Glu - Gly - Thr - Pro - Lys - Pro - Gln - Ser - His - Asn -
              55                    60
Asp - Gly - Asp - Phe - Glu - Glu - Ile - Pro - Glu - Glu -
              65
Tyr(R) - Leu - Gln
```

(A)

in welcher A Val, B Val, C Gln, E Asn, F Glu, G Gly, I Asn, J Lys, K Ile, L Asp, M Lys, N Gln und R Wasserstoff oder $SO_3H$ bedeuten,
oder
in welcher A Val, B Val, C Gln, E Asn, F Glu, G Gly, I Asn, J Asn, K Ile, L Asp, M Lys, N Gln und R Wasserstoff oder $SO_3H$ bedeuten,
oder
in welcher A Ile, B Thr, C Gln, E Asn, F Glu, G Gly, I Asn, J Asn, K Lys, L Asp, M Glu, N Gln und R Wasserstoff oder $SO_3H$ bedeuten,
oder
in welcher A Ile, B Thr, C Gln, E Asp, F Glu, G Gly, I Asn, J Lys, K Ile, L Asn, M Glu, N Gln und R Wasserstoff oder $SO_3H$ bedeuten,

27

oder

in welcher A Ile, B Thr, C Gln, E Asp, F Glu, G Gly, I Asp, J Lys, K Ile, L Asn, M Glu, N Gln und R Wasserstoff oder $SO_3H$ bedeuten,

oder

in welcher A Ile, B Thr, C Gln, E Asn, F Glu, G Gly, I Asn, J Lys, K Ile, L Asn, M Glu, N Gln und R Wasserstoff oder $SO_3H$ bedeuten,

oder

in welcher A Val, B Val, C Gln, E Asn, F Glu, G Asp, I Asn, J Glu, K Ile, L Asn, M Lys, N Gln und R Wasserstoff oder $SO_3H$ bedeuten,

oder

in welcher A Val, B Val, C Glu, E Asp, F Glu, G Gly, I Asn, J Glu, K Ile, L Asp, M Lys, N Glu und R Wasserstoff oder $SO_3H$ bedeuten,

oder

in welcher A Val, B Val, C Gln, E Asn, F Glu, G Asp, I Asn, J Gln, K Ile, L Asn, M Lys, N Gln und R Wasserstoff oder $SO_3H$ bedeuten,

oder

in welcher A Val, B Val, C Gln, E Asn, F Gln, G Asp, I Asn, J Gln, K Ile, L Asn, M Lys, N Gln und R Wasserstoff oder $SO_3H$ bedeuten,

oder

in welcher A Val, B Val, C Gln, E Asn, F Gln, G Gly, I Asn, J Gln, K Ile, L Asn, M Lys, N Gln und R Wasserstoff oder $SO_3H$ bedeuten.

2. Verfahren zur Herstellung eines Isohirudines, gemäß Anspruch 1, dadurch gekennzeichnet, daß man Isohirudin aus Blutegeln mit Hilfe einer Kombination von Extraktionsmethoden, Fällungsmethoden und chromatographischen Verfahren isoliert und reinigt, eine gegebenenfalls vorhandene Phenolestergruppe R gewünschtenfalls hydrolytisch unter Bildung der phenolischen Hydroxylgruppe abspaltet und das erhaltene Polypeptid gegebenenfalls in seine physiologisch verträglichen Salze überführt.

3. Isohirudin gemäß Anspruch 1 zur Anwendung als Heilmittel.

4. Isohirudin gemäß Anspruch 1 zur Anwendung als Thrombininhibitor.

5. Pharmazeutisches Mittel enthaltend ein Isohirudin gemäß Anspruch 1 und einen pharmazeutisch unbedenklichen Träger.

6. Verfahren zur Herstellung eines pharmazeutischen Mittels gemäß Anspruch 5, dadurch gekennzeichnet, daß man dieses und einen Träger in eine geeignete Darreichungsfrom bringt.

**Patentansprüche für folgende Vertragsstaaten : ES, GR**

1. Verfahren zur Herstellung eines Isohirudins der Formel (A)

$$
\begin{aligned}
&1\quad\quad\quad\quad 5\quad\quad\quad\quad\quad\quad\quad 10\\
&\text{A - B - Tyr - Thr - Asp - Cys - Thr - Glu - Ser - Gly -}\\
&\quad\quad\quad\quad\quad 15\quad\quad\quad\quad\quad 20\\
&\text{C - E - Leu - Cys - Leu - Cys - F - G - Ser - I - Val -}\\
&\quad\quad\quad\quad\quad 25\quad\quad\quad\quad\quad 30\\
&\text{Cys - Gly - J - Gly - Asn - Lys - Cys - K - Leu - Gly -}\\
&\quad\quad\quad\quad\quad 35\quad\quad\quad\quad\quad\quad 40\\
&\text{Ser - L - Gly - Glu - M - Asn - N - Cys - Val - Thr - Gly -}\\
&\quad\quad\quad\quad\quad 45\quad\quad\quad\quad\quad\quad 50\\
&\text{Glu - Gly - Thr - Pro - Lys - Pro - Gln - Ser - His - Asn -}\\
&\quad\quad\quad\quad\quad 55\quad\quad\quad\quad\quad\quad 60\\
&\text{Asp - Gly - Asp - Phe - Glu - Glu - Ile - Pro - Glu - Glu -}\\
&\quad\quad\quad\quad\quad 65\\
&\text{Tyr(R) - Leu - Gln}
\end{aligned}
$$

(A)

in welcher A Val, B Val, C Gln, E Asn, F Glu, G Gly, I Asn, J Lys, K Ile, L Asp, M Lys, N Gln und R Wasserstoff oder $SO_3H$ bedeuten, oder

in welcher A Val, B Val, C Gln, E Asn, F Glu, G Gly, I Asn, J Asn, K Ile, L Asp, M Lys, N Gln und R Wasserstoff oder $SO_3H$ bedeuten, oder

in welcher A Ile, B Thr, C Gln, E Asn, F Glu, G Gly, I Asn, J Asn, K Lys, L Asp, M Glu, N Gln und R Wasserstoff oder $SO_3H$ bedeuten, oder

in welcher A Ile, B Thr, C Gln, E Asp, F Glu, G Gly, I Asn, J Lys, K Ile, L Asn, M Glu, N Gln und R Wasserstoff oder $SO_3H$ bedeuten, oder

in welcher A Ile, B Thr, C Gln, E Asp, F Glu, G Gly, I Asp, J Lys, K Ile, L Asn, M Glu, N Gln und R Wasserstoff oder $SO_3H$ bedeuten, oder

in welcher A Ile, B Thr, C Gln, E Asn, F Glu, G Gly, I Asn, J Lys, K Ile, L Asn, M Glu, N Gln und R Wasserstoff oder $SO_3H$ bedeuten, oder

in welcher A Val, B Val, C Gln, E Asn, F Glu, G Asp, I Asn, J Glu, K Ile, L Asn, M Lys, N Gln und R Wasserstoff oder $SO_3H$ bedeuten, oder

in welcher A Val, B Val, C Glu, E Asp, F Glu, G Gly, I Asn, J Glu, K Ile, L Asp, M Lys, N Glu und R Wasserstoff oder $SO_3H$ bedeuten, oder

in welcher A Val, B Val, C Gln, E Asn, F Glu, G Asp, I Asn, J Gln, K Ile, L Asn, M Lys, N Gln und R Wasserstoff oder $SO_3H$ bedeuten, oder

in welcher A Val, B Val, C Gln, E Asn, F Gln, G Asp, I Asn, J Gln, K Ile, L Asn, M Lys, N Gln und R Wasserstoff oder $SO_3H$ bedeuten, oder

in welcher A Val, B Val, C Gln, E Asn, F Gln, G Gly, I Asn, J Gln, K Ile, L Asn, M Lys, N Gln und R Wasserstoff oder $SO_3H$ bedeuten,

29

dadurch gekennzeichnet, daß man Isohirudin aus Blutegeln mit Hilfe einer Kombination von Extraktionsmethoden, Fällungsmethoden und chromatographischen Verfahren isoliert und reinigt, eine gegebenenfalls vorhandene Phenolestergruppe R gewünschtenfalls hydrolytisch unter Bildung der phenolischen Hydroxylgruppe abspaltet und das erhaltene Polypeptid gegebenenfalls in seine physiologisch verträglichen Salze überführt.

2. Verwendung eines nach einem Verfahren gemäß Anspruch 1 hergestellten Isohirudins der Formel A als Thrombininhibitor.

3. Verfahren zur Herstellung einer pharmazeutischen Zubereitung enthaltend ein nach einem der Verfahren gemäß Anspruch 1 hergestellten Isohirudin der Formel A, dadurch gekennzeichnet, daß man dieses zusammen mit einem physiologisch unbedenklichen Träger und gegebenenfalls weiteren Hilfs- und Zusatzstoffen in eine geeignete Darreichungsform bringt.

**Claims**
**Claims for the following Contracting States : AT, BE, CH, DE, FR, GB, IT, LI, LU, NL, SE**

1. An isohirudin of the formula (A)

```
        1                    5                          10
       A - B - Tyr - Thr - Asp - Cys - Thr - Glu - Ser - Gly -

                        15                    20
       C - E - Leu - Cys - Leu - Cys - F - G - Ser - I - Val -

                        25                    30
       Cys - Gly - J - Gly - Asn - Lys - Cys - K - Leu - Gly -

                        35                    40
       Ser - L - Gly - Glu - M - Asn - N - Cys - Val - Thr - Gly -

                        45                    50
       Glu - Gly - Thr - Pro - Lys - Pro - Gln - Ser - His - Asn -

                        55                    60
       Asp - Gly - Asp - Phe - Glu - Glu - Ile - Pro - Glu - Glu -

                        65
       Tyr(R) - Leu - Gln                                        (A)
```

in which
A denotes Val, B denotes Val, C denotes Gln, E denotes Asn, F denotes Glu, G denotes Gly, I denotes Asn, J denotes Lys, K denotes Ile, L denotes Asp, M denotes Lys, N denotes Gln and R denotes hydrogen or $SO_3H$, or in which A denotes Val, B denotes Val, C denotes Gln, E denotes Asn, F denotes Glu, G denotes Gly, I denotes Asn, J denotes Asn, K denotes Ile, L denotes Asp, M denotes Lys, N denotes Gln and R denotes hydrogen or $SO_3H$, or in which A denotes Ile, B denotes Thr, C denotes Gln, E denotes Asn, F denotes Glu, G denotes Gly, I denotes Asn, J denotes Asn, K denotes Lys, L denotes Asp, M denotes Glu, N denotes Gln and R denotes hydrogen or $SO_3H$, or in which A denotes Ile, B denotes Thr, C denotes Gln, E denotes Asp, F denotes Glu, G denotes Gly, I denotes Asn, J denotes Lys, K denotes Ile, L denotes Asn, M denotes Glu, N denotes Gln and R denotes hydrogen or $SO_3H$, or in which A denotes Ile, B denotes Thr, C denotes Gln, E denotes Asp, F denotes Glu, G denotes Gly, I denotes Asp, J denotes Lys, K denotes Ile, L denotes Asn, M denotes Glu, N denotes Gln and R denotes hydrogen or $SO_3H$, or in which A denotes Ile, B denotes Thr, C denotes Gln, E denotes Asn, F denotes Glu, G denotes Gly, I denotes Asn, J denotes Lys, K denotes Ile, L denotes Asn, M denotes Glu, N denotes Gln and R denotes hydrogen or $SO_3H$, or in which A denotes Val, B denotes Val, C denotes Gln, E denotes Asn, F denotes Glu, G denotes Asp, I denotes Asn, J denotes Glu, K denotes Ile, L denotes Asn, M denotes Lys, N denotes Gln and R denotes hydrogen or $SO_3H$, or in which A denotes Val, B denotes Val, C denotes Glu, E denotes Asp, F denotes Glu, G denotes Gly, I denotes Asn, J. denotes Glu, K denotes lle, L denotes Asp, M denotes Lys, N denotes Glu and R denotes hydrogen or SO₃H, or in which A denotes Val, B denotes Val, C denotes Gln, E denotes Asn, F denotes Glu, G denotes Asp, I denotes Asn, J denotes Gln, K denotes lle, L denotes Asn, M denotes Lys, N denotes Gln and R denotes hydrogen or SO₃H, or in which A denotes Val, B denotes Val, C denotes Gln, E denotes Asn, F denotes Gln, G denotes Asp, I denotes Asn, J denotes Gln, K denotes lle, L denotes Asn, M denotes Lys, N denotes Gln and R denotes hydrogen or SO₃H, or in which A denotes Val, B denotes Val, C denotes Gln, E denotes Asn, F denotes Gln, G denotes Gly, I denotes Asn, J denotes Gln, K denotes lle, L denotes Asn, M denotes Lys, N denotes Gln and R denotes hydrogen or SO₃H.

2. A process for the preparation of an isohirudin as claimed in claim 1, which comprises isolating and purifying isohirudins from leeches with the aid of a combination of extraction methods, precipitation methods and chromatographic methods, if desired hydrolytically splitting off an optionally present phenol ester group R with the formation of the phenolic hydroxyl group and optionally converting the resulting polypeptide into its physiologically tolerable salts.

3. An isohirudin as claimed in claim 1 for use as a pharmaceutical.

4. An isohirudin as claimed in claim 1 for use as a thrombin inhibitor.

5. A pharmaceutical agent containing an isohirudin as claimed in claim 1 and a pharmaceutically acceptable excipient.

6. A process for the preparation of a pharmaceutical agent as claimed in claim 5, wherein the pharmaceutical agent and an excipient are brought into a suitable form for administration.

**Claims for the following Contracting States : ES, GR**

1. A process for the preparation of an isohirudin of the formula (A)

```
   1                5                          10
   A - B - Tyr - Thr - Asp - Cys - Thr - Glu - Ser - Gly -

                    15                         20
   C - E - Leu - Cys - Leu - Cys - F - G - Ser - I - Val -

                    25                         30
   Cys - Gly - J - Gly - Asn - Lys - Cys - K - Leu - Gly -

                    35                         40
   Ser - L - Gly - Glu - M - Asn - N - Cys - Val - Thr - Gly -

                45                         50
   Glu - Gly - Thr - Pro - Lys - Pro - Gln - Ser - His - Asn -

                55                         60
   Asp - Gly - Asp - Phe - Glu - Glu - Ile - Pro - Glu - Glu -

                65
   Tyr(R) - Leu - Gln                                       (A)
```

in which
A denotes Val, B denotes Val, C denotes Gln, E denotes Asn, F denotes Glu, G denotes Gly, I denotes Asn, J denotes Lys, K denotes lle, L denotes Asp, M denotes Lys, N denotes Gln and R denotes hydrogen or SO₃H, or in which A denotes Val, B denotes Val, C denotes Gln, E denotes Asn, F denotes Glu, G denotes Gly, I denotes Asn, J denotes Asn, K denotes lle, L denotes Asp, M denotes Lys, N denotes Gln and R denotes hydrogen or SO₃H, or in which A denotes lle, B denotes Thr, C denotes Gln, E denotes Asn, F denotes Glu, G denotes Gly, I denotes Asn, J denotes Asn, K denotes Lys, L denotes Asp, M denotes Glu, N denotes Gln and R denotes hydrogen or $SO_3H$, or in which A denotes Ile, B denotes Thr, C denotes Gln, E denotes Asp, F denotes Glu, G denotes Gly, I denotes Asn, J denotes Lys, K denotes Ile, L denotes Asn, M denotes Glu, N denotes Gln and R denotes hydrogen or $SO_3H$, or in which A denotes Ile, B denotes Thr, C denotes Gln, E denotes Asp, F denotes Glu, G denotes Gly, I denotes Asp, J denotes Lys, K denotes Ile, L denotes Asn, M denotes Glu, N denotes Gln and R denotes hydrogen or $SO_3H$, or in which A denotes Ile, B denotes Thr, C denotes Gln, E denotes Asn, F denotes Glu, G denotes Gly, I denotes Asn, J denotes Lys, K denotes Ile, L denotes Asn, M denotes Glu, N denotes Gln and R denotes hydrogen or $SO_3H$, or in which A denotes Val, B denotes Val, C denotes Gln, E denotes Asn, F denotes Glu, G denotes Asp, I denotes Asn, J denotes Glu, K denotes Ile, L denotes Asn, M denotes Lys, N denotes Gln and R denotes hydrogen or $SO_3H$, or in which A denotes Val, B denotes Val, C denotes Glu, E denotes Asp, F denotes Glu, G denotes Gly, I denotes Asn, J. denotes Glu, K denotes Ile, L denotes Asp, M denotes Lys, N denotes Glu and R denotes hydrogen or $SO_3H$, or in which A denotes Val, B denotes Val, C denotes Gln, E denotes Asn, F denotes Glu, G denotes Asp, I denotes Asn, J denotes Gln, K denotes Ile, L denotes Asn, M denotes Lys, N denotes Gln and R denotes hydrogen or $SO_3H$, or in which A denotes Val, B denotes Val, C denotes Gln, E denotes Asn, F denotes Gln, G denotes Asp, I denotes Asn, J denotes Gln, K denotes Ile, L denotes Asn, M denotes Lys, N denotes Gln and R denotes hydrogen or $SO_3H$, or in which A denotes Val, B denotes Val, C denotes Gln, E denotes Asn, F denotes Gln, G denotes Gly, I denotes Asn, J denotes Gln, K denotes Ile, L denotes Asn, M denotes Lys, N denotes Gln and R denotes hydrogen or $SO_3H$, which comprises isolating and purifying isohirudins from leeches with the aid of a combination of extraction methods, precipitation methods and chromatographic methods, if desired hydrolytically splitting off an optionally present phenol ester group R with the formation of the phenolic hydroxyl group and optionally converting the resulting polypeptide into its physiologically tolerable salts.

2. The use of an isohirudin of the formula (A) prepared by a process as claimed in claim 1 as a thrombin inhibitor.

3. A process for the preparation of a pharmaceutical preparation containing an isohirudin of the formula A prepared by a process as claimed in claim 1, which comprises bringing the isohirudin, together with a physiologically acceptable excipient and, if desired, further auxiliaries and adjuvants, into a suitable form for administration.

**Revendications**

**Revendications pour les Etats contractants suivants : AT, BE, CH, DE, FR, GB, IT, LI, LU, NL, SE**

1. Isohirudine de formule (A)

```
     1                 5                          10
A - B - Tyr - Thr - Asp - Cys - Thr - Glu - Ser - Gly -

                       15                        20
C - E - Leu - Cys - Leu - Cys - F - G - Ser - I - Val -

                       25                        30
Cys - Gly - J - Gly - Asn - Lys - Cys - K - Leu - Gly -

                       35                        40
Ser - L - Gly - Glu - M - Asn - N - Cys - Val - Thr - Gly -        (A)

                       45                        50
Glu - Gly - Thr - Pro - Lys - Pro - Gln - Ser - His - Asn -

                       55                        60
Asp - Gly - Asp - Phe - Glu - Glu - Ile - Pro - Glu - Glu -

                       65
Tyr(R) - Leu - Gln
```

dans laquelle A représente Val, B Val, C Gln, E Asn, F Glu, G Gly, I Asn, J Lys, K Ile, L Asp, M Lys, N Gln et R un atome d'hydrogène ou SO$_3$H,

ou

dans laquelle A représente Val, B Val, C Gln, E Asn, F Glu, G Gly, I Asn, J Asn, K Ile, L Asp, M Lys, N Gln et R un atome d'hydrogène ou SO$_3$H,

ou

dans laquelle A représente Ile, B Thr, C Gln, E Asn, F Glu, G Gly, I Asn, J Asn, K Lys, L Asp, M Glu, N Gln et R un atome d'hydrogène ou SO$_3$H,

ou

dans laquelle A représente Ile, B Thr, C Gln, E Asp, F Glu, G Gly, I Asn, J Lys, K Ile, L Asn, M Glu, N Gln et R un atome d'hydrogène ou SO$_3$H,

ou

dans laquelle A représente Ile, B Thr, C Gln, E Asp, F Glu, G Gly, I Asp, J Lys, K Ile, L Asn, M Glu, N Gln et R un atome d'hydrogène ou SO$_3$H,

ou

dans laquelle A représente Ile, B Thr, C Gln, E Asn, F Glu, G Gly, I Asn, J Lys, K Ile, L Asn, M Glu, N Gln et R un atome d'hydrogène ou SO$_3$H,

ou

dans laquelle A représente Val, B Val, C Gln, E Asn, F Glu, G Asp, I Asn, J Glu, K Ile, L Asn, M Lys, N Gln et R un atome d'hydrogène ou SO$_3$H,

ou

dans laquelle A représente Val, B Val, C Glu, E Asp, F Glu, G Gly, I Asn, J Glu, K Ile, L Asp, M Lys, N Glu et R un atome d'hydrogène ou SO$_3$H,

ou

dans laquelle A représente Val, B Val, C Gln, E Asn, F Glu, G Asp, I Asn, J Gln, K Ile, L Asn, M Lys, N Gln et R un atome d'hydrogène ou SO$_3$H,

ou

dans laquelle A représente Val, B Val, C Gln, E Asn, F Gln, G Asp, I Asn, J Gln, K Ile, L Asn, M Lys, N Gln et R un atome d'hydrogène ou SO$_3$H,

ou

dans laquelle A représente Val, B Val, C Gln, E Asn, F Gln, G Gly, I Asn, J Gln, K Ile, L Asn, M Lys, N Gln et R un atome d'hydrogène ou SO$_3$H.

2. Procédé pour la préparation d'une isohirudine selon la revendication 1, caractérisé en ce que l'on isole et purifie de l'isohuridine à partir de sangsues, à l'aide d'une combinaison de méthodes d'extraction, de méthodes de précipitation et de procédés chromatographiques, et, si on le désire, on élimine par hydrolyse un groupe ester phénolique R éventuellement présent, avec formation du groupe hydroxy phénolique, et éventuellement on convertit le polypeptide obtenu en ses sels physiologiquement acceptables.

3. Isohirudine selon la revendication 1, pour utilisation en tant que médicament.

4. Isohirudine selon la revendication 1, pour utilisation en tant qu'inhibiteur de la thrombine.

5. Composition pharmaceutique contenant une isohirudine selon la revendication 1 et un véhicule pharmaceutiquement acceptable.

6. Procédé pour la préparation d'une composition pharmaceutique selon la revendication 5, caractérisé en ce qu'on met sous une forme d'administration appropriée cette isohirudine et un véhicule.

EP 0 364 942 B1

**Revendications pour les Etats contractants suivants : ES, GR**

1.  Procédé pour la préparation d'une isohirudine de formule (A)

$$
\begin{array}{l}
\;\;\;\;1\;\;\;\;\;\;\;\;\;\;\;\;\;\;\;\;\;\;5\;\;\;\;\;\;\;\;\;\;\;\;\;\;\;\;\;\;\;\;\;\;\;\;\;\;\;\;\;\;\;10 \\
A - B - Tyr - Thr - Asp - Cys - Thr - Glu - Ser - Gly - \\[4pt]
\;\;\;\;\;\;\;\;\;\;\;\;\;\;\;\;\;\;\;\;\;\;15\;\;\;\;\;\;\;\;\;\;\;\;\;\;\;\;\;\;\;\;\;\;20 \\
C - E - Leu - Cys - Leu - Cys - F - G - Ser - I - Val - \\[4pt]
\;\;\;\;\;\;\;\;\;\;\;\;\;\;\;\;\;\;\;25\;\;\;\;\;\;\;\;\;\;\;\;\;\;\;\;\;\;\;\;\;\;30 \\
Cys - Gly - J - Gly - Asn - Lys - Cys - K - Leu - Gly - \\[4pt]
\;\;\;\;\;\;\;\;\;\;\;\;\;\;\;\;\;\;\;35\;\;\;\;\;\;\;\;\;\;\;\;\;\;\;\;\;\;\;\;\;\;40 \\
Ser - L - Gly - Glu - M - Asn - N - Cys - Val - Thr - Gly - \\[4pt]
\;\;\;\;\;\;\;\;\;\;\;\;\;\;\;\;\;\;\;45\;\;\;\;\;\;\;\;\;\;\;\;\;\;\;\;\;\;\;\;\;\;50 \\
Glu - Gly - Thr - Pro - Lys - Pro - Gln - Ser - His - Asn - \\[4pt]
\;\;\;\;\;\;\;\;\;\;\;\;\;\;\;\;\;\;\;55\;\;\;\;\;\;\;\;\;\;\;\;\;\;\;\;\;\;\;\;\;\;60 \\
Asp - Gly - Asp - Phe - Glu - Glu - Ile - Pro - Glu - Glu - \\[4pt]
\;\;\;\;\;\;\;\;\;\;\;\;\;\;\;\;\;\;\;65 \\
Tyr(R) - Leu - Gln
\end{array}
\qquad (A)
$$

dans laquelle A représente Val, B Val, C Gln, E Asn, F Glu, G Gly, I Asn, J Lys, K Ile, L Asp, M Lys, N Gln et R un atome d'hydrogène ou $SO_3H$,
ou
dans laquelle A représente Val, B Val, C Gln, E Asn, F Glu, G Gly, I Asn, J Asn, K Ile, L Asp, M Lys, N Gln et R un atome d'hydrogène ou $SO_3H$,
ou
dans laquelle A représente Ile, B Thr, C Gln, E Asn, F Glu, G Gly, I Asn, J Asn, K Lys, L Asp, M Glu, N Gln et R un atome d'hydrogène ou $SO_3H$,
ou
dans laquelle A représente Ile, B Thr, C Gln, E Asp, F Glu, G Gly, I Asn, J Lys, K Ile, L Asn, M Glu, N Gln et R un atome d'hydrogène ou $SO_3H$,
ou
dans laquelle A représente Ile, B Thr, C Gln, E Asp, F Glu, G Gly, I Asp, J Lys, K Ile, L Asn, M Glu, N Gln et R un atome d'hydrogène ou $SO_3H$,
ou
dans laquelle A représente Ile, B Thr, C Gln, E Asn, F Glu, G Gly, I Asn, J Lys, K Ile, L Asn, M Glu, N Gln et R un atome d'hydrogène ou $SO_3H$,
ou
dans laquelle A représente Val, B Val, C Gln, E Asn, F Glu, G Asp, I Asn, J Glu, K Ile, L Asn, M Lys, N Gln et R un atome d'hydrogène ou $SO_3H$,
ou
dans laquelle A représente Val, B Val, C Glu, E Asp, F Glu, G Gly, I Asn, J Glu, K Ile, L Asp, M Lys, N Glu et R un atome d'hydrogène ou $SO_3H$,
ou
dans laquelle A représente Val, B Val, C Gln, E Asn, F Glu, G Asp, I Asn, J Gln, K Ile, L Asn, M Lys, N Gln et R un atome d'hydrogène ou $SO_3H$,
ou
dans laquelle A représente Val, B Val, C Gln, E Asn, F Gln, G Asp, I Asn, J Gln, K Ile, L Asn, M Lys, N Gln et R un atome d'hydrogène ou $SO_3H$,
ou
dans laquelle A représente Val, B Val, C Gln, E Asn, F Gln, G Gly, I Asn, J Gln, K Ile, L Asn, M Lys, N Gln et R un atome d'hydrogène ou $SO_3H$,
caractérisé en ce que l'on isole et purifie de l'isohuridine à partir de sangsues, à l'aide d'une

34

combinaison de méthodes d'extraction, de méthodes de précipitation et de procédés chromatographiques, et, si on le désire, on élimine par hydrolyse un groupe ester phénolique R éventuellement présent, avec formation du groupe hydroxy phénolique, et éventuellement on convertit le polypeptide obtenu en ses sels physiologiquement acceptables.

2. Utilisation d'une isohirudine de formule A, préparée par un procédé selon la revendication 1, en tant qu'inhibiteur de la thrombine.

3. Procédé pour la préparation d'une composition pharmaceutique contenant une isohirudine de formule A, préparée par un procédé selon la revendication 1, caractérisé en ce qu'on met sous une forme d'administration appropriée cette isohirudine, conjointement avec un véhicule physiologiquement acceptable et éventuellement d'autres adjuvants et additifs.